# EUROPÄISCHE PATENTSCHRIFT

(11) **EP 4 149 370 B1**
(45) Veröffentlichungstag und Bekanntmachung des Hinweises auf die Patenterteilung: **10.12.2025**
(21) Anmeldenummer: 21724675.0
(22) Anmeldetag: 10.05.2021
(51) Int. Cl.: A61B 17/122, A61B 90/92

(54) **MEDIZINISCHER CLIP UND VERFAHREN ZUM HERSTELLEN EINES MEDIZINISCHEN IMPLANTATS**
MEDICAL CLIP AND METHOD FOR PRODUCING A MEDICAL IMPLANT
CLIP MÉDICAL ET PROCÉDÉ DE PRODUCTION D'UN IMPLANT MÉDICAL

(30) Priorität: 12.05.2020 DE 102020112781
(43) Veröffentlichungstag der Anmeldung: 22.03.2023
(73) Patentinhaber: Aesculap AG, 78532 Tuttlingen (DE)
(72) Erfinder: HAPPLE, Alexander, 78183 Hüfingen (DE); ALLERBORN, Andreas, 78194 Immendingen (DE); BEGER, Jens, 78532 Tuttlingen (DE)
(74) Vertreter: Hoeger, Stellrecht & Partner Patentanwälte mbB
(86) Internationale Anmeldenummer: PCT/EP2021/062258
(87) Internationale Veröffentlichungsnummer: WO 2021/228736

(56) Entgegenhaltungen:
- EP-A1- 2 457 530
- WO-A1-2007/124579
- CN-U- 203 576 581
- US-A- 4 765 335
- US-A- 5 797 931
- US-A1- 2008 097 442

## Beschreibung

Die vorliegende Erfindung betrifft einen medizinischen Clip, insbesondere in Form eines Aneurysmenclips, welcher zwei Klemmarme und ein vorspannendes Element umfasst, wobei jeweils ein Klemmarm an einem freien Ende des vorspannenden Elements angeordnet oder ausgebildet ist und wobei die Klemmarme in einer Grundstellung aneinander anliegen und entgegen der Wirkung des vorspannenden Elements in eine Öffnungsstellung voneinander weg bewegbar sind, wobei mindestens ein Teil einer Oberfläche des Clips farbig ausgebildet ist, wobei der Clip mindestens drei voneinander räumlich getrennte Clipbereiche definiert und benachbarte Clipbereiche der mindestens drei Clipbereiche unterschiedlich farbig ausgebildet sind.

Ferner betrifft die vorliegende Erfindung ein medizinisches Clipsystem umfassend mindestens zwei medizinische Clips, wobei mindestens ein Clip in Form eines permanenten Clips ausgebildet ist, dessen Oberfläche farbig, insbesondere vollständig, ausgebildet ist, und wobei mindestens ein Clip, welcher in Form und/oder Größe dem permanenten Clip entspricht, in Form einen temporären Clips ausgebildet ist, dessen Oberfläche mindestens teilweise farbig entsprechend dem permanenten Clip und mindestens teilweise farbig zur Kodierung als temporärer Clip ausgebildet ist.

Überdies betrifft die vorliegende Erfindung ein Verfahren zum Herstellen eines medizinischen Implantats, nämlich eines medizinischen Clips, wobei eine Implantatoberfläche des medizinischen Implantats farbig ausgebildet wird, wobei am Implantat mindestens drei voneinander räumlich getrennte Implantatbereiche definiert werden und benachbarte Implantatbereiche der mindestens drei Implantatbereiche unterschiedlich farbig ausgebildet werden.

Clipsysteme der eingangs beschriebenen Art sind insbesondere in Form von Aneurysmenclip-Systemen bekannt. Dabei sind sogenannte permanente Clips vollständig einfarbig ausgebildet. Die Farbe dient dabei insbesondere zur Kodierung der Form und/oder der Größe des permanenten Clips.

Vor der endgültigen Platzierung eines solchen permanenten Clips, welcher bestimmungsgemäß zum dauerhaften Verbleib im Körper eines Patienten bestimmt ist, werden während eines chirurgischen Eingriffs, beispielsweise zur Behandlung eines Aneurysmas, sogenannte temporäre Clips eingesetzt. Diese sind teilweise farbig korrespondierend zum permanenten Clip, den sie temporär ersetzen, eingefärbt, und teilweise farbig als eine Kodierung derart, dass es sich bei diesem Clip um einen temporären Clip handelt. Eine solche farbige Gestaltung beziehungsweise Kodierung temporärer Clips ermöglicht es einem Operateur, zum einen zwischen Form und/oder Größe der Clips zu unterscheiden und ihm zudem eine Unterscheidung zwischen temporären Clips und permanenten Clips zu ermöglichen.

Ein Problem bei derartigen Clipsystemen ist insbesondere, dass für einen Operateur beispielsweise bei einem minimalinvasiven Eingriff nicht immer eindeutig erkennbar ist, ob es sich um einen temporären Clip oder um einen permanenten Clip handelt. Dies kann beispielsweise daran liegen, dass ein Teil des Clips, der in einer Farbe gehalten ist, welche den Clip als temporären Clip kodiert, von einem Einsetzinstrument verdeckt ist. Dadurch kann es in unerwünschter Weise zu Verwechslungen von temporären Clips und permanenten Clips kommen.

Aus der WO 2007/124579 A1 sind ein chirurgischer Clip, ein Applikator und Applikationsverfahren bekannt. Ein medizinisches Implantat ist in der EP 2 457 530 A1 beschrieben. Die US 2008/0097442 A1 betrifft Verfahren und Vorrichtungen zum Verbessern der Funktion von sich verjüngenden Verriegelungen, die für die Wirbelsäulenstabilisierung verwendet werden. Ein Aneurysmenclip ist aus der US 4,765,335 bekannt. In der CN 203 576 581 U ist ein Tumorclip beschrieben, wobei dessen Länge mit Farben angezeigt wird. Ein chirurgisches Instrument zum Befestigen von Gewebe, eine Vorrichtung zum Befestigen von Gewebe sowie Verfahren zum Befestigen von Gewebe sind in der US 5,797,931 offenbart.

Es ist daher eine Aufgabe der vorliegenden Erfindung, einen medizinischen Clip, ein medizinisches Clipsystem und ein Verfahren der eingangs beschriebenen Art so zu verbessern, dass eine sichere Unterscheidung der Clips ermöglicht wird.

Diese Aufgabe wird bei einem medizinischen Clip der eingangs beschriebenen Art erfindungsgemäß dadurch gelöst, dass ein erster Clipbereich der mindestens drei Clipbereiche freie Enden der zwei Klemmarme umfasst, dass ein zweiter Clipbereich der mindestens drei Clipbereiche das vorspannende Element oder einen Teil desselben umfasst und dass mindestens ein dritter Clipbereich der mindestens drei Clipbereiche zwischen dem ersten Clipbereich und dem zweiten Clipbereich angeordnet oder ausgebildet ist.

Einen bekannten Clip, welcher ein konkretes Ausführungsbeispiel eines medizinischen Implantats ist, in der beschriebenen Weise weiterzubilden hat beispielsweise den Vorteil, dass ein solcher medizinischer Clip insbesondere einfach und sicher identifiziert werden kann, beispielsweise als temporärer Clip. Eine solche Identifizierung kann insbesondere auch mit hoher Zuverlässigkeit bei minimalinvasiven chirurgischen Eingriffen, unter dem Mikroskop oder auch beim Applizieren eines solchen Clips, wenn dieser beispielsweise im Maul eines Applikationswerkzeugs aufgenommen und dadurch teilweise verdeckt wird, sichergestellt werden. Beispielsweise können zwei der drei Clipbereiche durch erste und zweite Endbereiche des Clips definiert werden. Wird beispielsweise ein Ende beziehungsweise ein Endbereich des ein medizinisches Implantat bildenden Clips mit einem Applikationswerkzeug erfasst und verdeckt, ist immer noch das andere Ende beziehungsweise der andere Endbereich des Clips frei sichtbar. Daher kann ein Anwender unabhängig davon, ob der Clip teilweise verdeckt ist oder nicht, mit hoher Zuverlässigkeit erkennen, ob es sich um einen temporären Clip oder um einen permanenten Clip handelt. Ferner kann auch eine eindeutige Unterscheidbarkeit von Clips auf einem Operationstisch oder in einem Lagerungssiebkorb erreicht werden, insbesondere auch in abgedunkelten Räumen wie insbesondere einem abgedunkelten Operationssaal. Ferner sei angemerkt, dass es sich bei dem medizinischen Clip um ein medizinisches Implantat handelt, welches statt in Form eines medizinischen Clips, insbesondere in Form eines Aneurysmenclips, nicht erfindungsgemäß auch in Form einer medizinischen Schraube, beispielsweise in Form einer Knochenschraube oder einer Pedikelschraube ausgebildet sein kann. Ein solches medizinisches Implantat weist eine Implantatoberfläche auf, wobei mindestens ein Teil der Implantatoberfläche farbig ausgebildet ist, wobei am Implantat mindestens drei voneinander räumlich getrennte Implantatbereiche definiert sind und wobei benachbarte Implantatbereiche der mindestens drei Implantatbereiche unterschiedlich farbig ausgebildet sind. Die Offenbarung ist nicht beschränkt auf medizinische Implantate in Form von medizinischen Clips, sondern umfasst beliebige Implantate, insbesondere in Form medizinischer Schrauben. Ferner ist farbig ausgebildet insbesondere so zu verstehen, dass die mindestens drei Clipbereiche beziehungsweise Implantatbereiche durchgefärbt sind, also aus einem farbigen Werkstoff ausgebildet sind, oder nur eine farbige Oberfläche aufweisen, die beispielsweise durch eine Beschichtung einen farbigen Eindruck beim Betrachter hinterlässt. So kann ein Werkstoff, aus dem das Material ausgebildet ist, andersfarbig sein als eine Implantatoberfläche in den mindestens drei Implantatbereichen. Zudem ist benachbart im Sinne der Ansprüche so zu verstehen, dass die benachbarten, unterschiedlich farbigen Implantatbereiche beziehungsweise Clipbereiche aneinander angrenzen, wobei sich die benachbarten Bereiche vorzugsweise nicht überlappen, sondern ohne Abstand oder mit nur einem geringen Abstand nebeneinander angeordnet oder ausgebildet sind. Ein Implantatbereich beziehungsweise ein Clipbereich kann insbesondere einen beliebigen Abschnitt des Implantats beziehungsweise des Clips bilden. Günstig ist es, dass ein erster Clipbereich der mindestens drei Clipbereiche freie Enden der zwei Cliparme umfasst, dass ein zweiter Clipbereich der mindestens zwei Clipbereiche das vorspannende Element oder einen Teil desselben umfasst und dass mindestens ein dritter Clipbereich der mindestens zwei Clipbereiche zwischen dem ersten Clipbereich und dem zweiten Clipbereich angeordnet oder ausgebildet ist. Diese vorgeschlagene Weiterbildung ermöglicht es insbesondere, einen drei, vier, fünf oder mehr Clipbereiche umfassenden Clip auszubilden, wobei die Clipbereiche unterschiedlich geformt sind. Beispielsweise kann so ein drei, vier, fünf oder noch mehr durch die Clipbereiche definierte Streifen aufweisender Clip ausgebildet werden. Insbesondere kann durch eine Anzahl der unterschiedlichen Clipbereiche eine Wahrscheinlichkeit minimiert werden, dass alle Clipbereiche, die zur Kennzeichnung des Clips als temporärer Clip dienen, gleichzeitig verdeckt werden können. Mit anderen Worten kann so mit einer sehr großen Wahrscheinlichkeit mindestens immer einer der Clipbereiche, der den Clip als temporären Clip kennzeichnet, für einen Anwender sichtbar bleiben.

Vorteilhaft ist es, wenn mindestens zwei der mindestens drei Clipbereiche gleichfarbig ausgebildet sind und wenn diese mindestens zwei Clipbereiche nicht direkt aneinander angrenzen. Diese Ausgestaltung ermöglicht es insbesondere, an einem Implantat voneinander beabstandete Enden in derselben Farbe zu gestalten. Wird beispielsweise ein Ende mit einem Applikationswerkzeug gefasst und gehalten, ist dann noch immer ein zweites Ende des Implantats für den Operateur oder einen anderen Anwender sichtbar und ermöglicht eine sichere und zuverlässige Unterscheidung eines solchen Clips beispielsweise von einem einfarbig ausgebildeten Clip.

Vorzugsweise sind der erste Clipbereich und der zweite Clipbereich heller gefärbt als der mindestens eine dritte Clipbereich. So können insbesondere im Operationssitus derartige Clips sicher identifiziert werden, beispielsweise als temporäre Clips, die nicht für eine dauerhafte Implantation vorgesehen sind.

Günstigerweise ist mindestens einer der mindestens drei Clipbereiche in einer der Farben Grün, Blau, Violett, Gelb oder goldfarbig gefärbt. Beispielsweise können die Farben Grün, Blau und Violett genutzt werden, um Form und/oder Größe des Clips zu kennzeichnen, gelbe oder goldfarbige Clipbereiche temporäre Clips zu kennzeichnen, um diese von permanenten Clips, also dauerhaft zu implantierenden Clips, unterscheiden zu können.

Vorteilhaft ist es, wenn entweder die Oberfläche des mindestens einen dritten Clipbereichs gelb oder goldfarbig ausgebildet ist oder wenn die Oberflächen des ersten Clipbereichs und des zweiten Clipbereichs gelb oder goldfarbig ausgebildet sind. Auf diese Weise kann insbesondere erreicht werden, dass mindestens ein Clipbereich der gelb oder goldfarbig ist, sichtbar bleibt, auch wenn der Clip an einem Ende, also beispielsweise am zweiten Clipbereich, gefasst und durch ein Applikationsinstrument verdeckt wird.

Gemäß einer weiteren bevorzugten Ausführungsform der Erfindung kann vorgesehen sein, dass zwei aneinander angrenzende Clipbereiche der mindestens drei Clipbereiche einen farbigen Übergangsbereich der Oberfläche definieren und dass der farbige Übergangsbereich an einem geometrisch definierten Bereich des Clips angeordnet oder ausgebildet ist. Insbesondere kann es sich beim farbigen Übergangsbereich um einen geometrisch definierten Bereich des Clips handeln, dessen Querschnitt auf einer Bereichslänge, welche mindestens etwa 5%, insbesondere etwa 10%, einer Gesamtlänge des Clips entspricht, konstant ist oder sich konisch verjüngt oder erweitert. Der farbige Übergangsbereich kann im Vergleich zur Gesamtlänge des Clips insbesondere sehr viel kürzer sein als die Bereichslänge. Beispielsweise können aneinander angrenzende Clipbereiche Farbübergänge in Form einer Stufenfunktion aufweisen, also eine mehr oder weniger schlagartige Farbänderung in räumlich unmittelbarer Nähe. Durch Vorsehen der Übergangsbereiche an einem geometrisch definierten Bereich des Clips können insbesondere definierte Farbübergänge erreicht werden. Insbesondere kann der geometrisch definierte Bereich vorgegebenen sein durch einen Bereich oder Abschnitt des Implantats, in dem ein Querschnitt konstant ist. Dies kann insbesondere im Bereich der Klemmarme sein. Geometrisch definierte Bereiche sind beispielsweise auch zylindrische Abschnitte oder Bereiche mit konstantem Querschnitt oder konische Abschnitte oder Bereiche, in denen ein Querschnitt zu- oder abnimmt, beispielsweise linear. Derartige Bereiche können beispielsweise im Bereich eines freien Endes des vorspannenden Elements ausgebildet sein. Insbesondere lässt sich so eine Herstellung der unterschiedlich farbigen Clipbereiche verbessern, was zu eindeutigeren Einfärbungen der unterschiedlichen Clipbereiche führt.

Günstig ist es, wenn die Oberfläche mindestens eines Clipbereichs der mindestens drei Clipbereiche durch eine Beschichtung farbig ausgebildet ist. Insbesondere können alle Clipbereiche durch eine Beschichtung farbig ausgebildet sein. Es kann sich dabei insbesondere um unterschiedliche Arten von Beschichtungen handeln. Beispielsweise kann ein Clipbereich durch anodische Oxidation ausgebildet werden. Ein zweiter und/oder dritter Clipbereich kann beispielsweise durch eine Beschichtung mit einer metallischen Beschichtung mittels eines Tauchverfahrens ausgebildet werden. Insbesondere können auch mehrere Schichten von Beschichtungen übereinander angeordnet sein, beispielsweise in einem oder mehreren Clipbereichen. Insbesondere kann der gesamte Clip durch eine anodische Beschichtung farbig ausgebildet werden. Ein zweiter und/oder dritter Clipbereich kann dann durch zusätzliche Beschichtung in einem Tauchverfahren mit einer metallischen Beschichtung versehen werden oder durch eine weitere anodische Oxidation.

Vorzugsweise liegt eine Schichtdicke der Beschichtung in einem Bereich von etwa 10 nm bis etwa 500 nm. Insbesondere liegt die Schichtdicke in einem Bereich von etwa 20 nm bis etwa 200 nm. Derartige Schichtdicken ermöglichen es insbesondere, zur Farbgebung Interferenzeffekte zu nutzen, beispielsweise wenn die Beschichtung in Form einer Oxidschicht ausgebildet ist. Beispielsweise kann eine Titandioxidschicht durch anodische Oxdation auf einem aus Titan ausgebildeten Clip in der beschriebenen Weise ausgebildet werden. Unterschiedliche Schichtdicken führen beispielsweise zu unterschiedlichen Farben. Auch bei einer Tauchbeschichtung kann die Schichtdicke in den angegebenen Bereichen liegen. So kann insbesondere mit einem minimalen Materialaufwand, was beispielsweise für Goldbeschichtungen vorteilhaft ist, eine sichere und zuverlässige dauerhafte Beschichtung des Clips in einem oder mehreren Clipbereichen erreicht werden.

Auf einfache und kostengünstige Weise lässt sich der Clip ausbilden, wenn die Beschichtung in Form einer durch anodische Oxidation ausgebildeten Oxidschicht ausgebildet ist. Insbesondere kann durch entsprechende Einstellung von Verfahrensparametern bei der anodischen Oxidation eine Farbe der Oxidschicht in gewünschter und definierter Weise vorgegeben werden.

Ferner kann es günstig sein, wenn die Oxidschicht einen Interferenzfilter bildet und wenn eine Farbwirkung der Oxidschicht von einer Schichtdicke derselben abhängt. So können insbesondere sehr dauerhafte, stabile farbige Beschichtungen von Clips ausgebildet werden. Durch entsprechende Verfahrensparameter beim anodischen Oxidieren können gewünschte Schichtdicken der jeweiligen Oxidschicht vorgegeben werden.

Die Herstellung des Clips lässt sich weiter vereinfachen, wenn Clipbereiche mit gleichfarbig ausgebildeter Oberfläche mit einer identischen Beschichtung versehen sind. So können insbesondere zwei oder mehr Clipbereiche in einem Verfahrensschritt mit identischen Beschichtungen versehen werden.

Vorzugsweise enthält die Beschichtung Gold oder besteht aus Gold. Eine solche Beschichtung kann insbesondere auf Werkstoffen zur Ausbildung medizinischer Clips eingesetzt werden, die nicht für eine anodische Oxidation geeignet sind, beispielsweise Clips, die aus Kobalt-Chrom-Legierungen, wie insbesondere Phynox, ausgebildet sind. Beispielsweise können beide Endbereiche des Clips, also insbesondere freie Ende der Klemmarme einerseits und das vorspannende Element andererseits, in einem Tauchverfahren mit Gold beschichtet werden.

Auf einfache Weise lässt sich der medizinische Clip beschichten, wenn die Beschichtung in Form einer Tauchbeschichtung ausgebildet ist. Beispielsweise können so Goldbeschichtungen auf nicht oxidierbaren Werkstoffen realisiert werden.

Ferner ist es vorteilhaft, wenn mindestens ein Clipbereich der mindestens drei Clipbereiche unbeschichtet ist. Ein solcher Clipbereich weist dann die Farbe des Werkstoff auf, aus dem der Clip gebildet ist. Beispielsweise kann dieser Werkstoff eine natürliche Oxidschicht aufweisen, wie beispielsweise Aluminium, das durch eine passivierende Aluminiumoxidschicht gegen Korrosion geschützt ist.

Vorzugsweise ist der Clip aus einem körperverträglichen Metall ausgebildet. So können insbesondere Abstoßungsreaktionen des Clips nach einer Implantation minimiert werden. Das körperverträgliche Metall kann insbesondere Titan, Aluminium oder Tantal sein. Ferner kann es sich auch um eine metallische Legierung handeln.

Vorzugsweise enthält die metallische Legierung Kobalt und/oder Chrom und/oder Nickel. Die genannten Metalle können in entsprechender Kombination insbesondere zur Ausbildung implantierbarer körperverträglicher Implantate genutzt werden. Beispielsweise kann es sich bei einer solchen Legierung um Phynox handeln.

Die eingangs gestellte Aufgabe wird ferner bei einem medizinischen Clipsystem der eingangs beschriebenen Art erfindungsgemäß dadurch gelöst, dass der temporäre Clip in Form einer der oben beschriebenen bevorzugten Ausführungsformen medizinischer Clips ausgebildet ist.

Ein wie vorgeschlagen weitergebildetes Clipsystem ermöglicht eine sichere Unterscheidung temporärer Clips und permanenter Clip. Für weitere Vorteile wird auf die obige Beschreibung verwiesen.

Günstig ist es, wenn die Farbe der Oberfläche des permanenten Clips eine Kodierung für Form und/oder Größe des permanenten Clips bildet. So kann ein Anwender Form und/oder Größe permanenter Clips direkt an deren Farbe oder Färbung erkennen. Beispielsweise kann so auch eine einfache Zuordnung zur jeweiligen Form/Größe korrespondierender Applikationsinstrumente erreicht werden, wenn diese mit einer korrespondierenden Kodierung versehen sind, beispielsweise vollständig oder teilweise in der Farbe des permanenten Clips eingefärbt sind.

Vorteilhaft ist es, wenn jeder der mindestens zwei medizinischen Clips zwei Klemmarme und ein vorspannendes Element umfasst, wobei jeweils ein Klemmarm an einem freien Ende des vorspannenden Elements angeordnet oder ausgebildet ist und wobei die zwei Klemmarme in einer Grundstellung aneinander anliegen und entgegen der Wirkung des vorspannenden Elements in eine Öffnungsstellung voneinander weg bewegbar sind. Mit derartigen Clips können insbesondere Aneurysmen oder andere Aussackungen an Hohlorganen in definierter Weise behandelt werden, beispielsweise durch Abklemmen.

Vorzugsweise sind der der permanente Clip und der zugeordnete temporäre Clip bis auf die Farbgebung identisch ausgebildet. So kann ein Anwender einen temporären Clip temporär einsetzen, bis der permanente Clip platziert wird und dann dauerhaft im Patienten verbleibt. So kann ein Operateur zunächst unterschiedliche temporäre Clips einsetzen, um für den jeweiligen Eingriff den optimalen Clip, also Form und Größe desselben, auszuwählen. Nach seiner endgültigen Auswahl kann er dann den temporären Clip durch den permanenten Clip ersetzen. Durch die wie vorgeschlagen ausgebildete farbliche Kennzeichnung der Clips kann dann ein Operateur jeweils sicher unterscheiden, ob ein eingesetzter Clip in Form eines temporären oder in Form eines permanenten Clips ausgebildet ist.

Vorteilhafterweise umfasst das Clipsystem mindestens zwei sich in Größe und/oder Form unterscheidende permanente Clips. Dies ermöglicht es einem Anwender, für unterschiedlichste Indikationen den passenden Clip verfügbar zu haben. Das Clipsystem kann insbesondere fünf, 10, 20 oder mehr unterschiedliche Formen und entsprechende Größen von Clips umfassen.

Die eingangs gestellte Aufgabe wird ferner bei einem Verfahren der eingangs beschriebenen Art erfindungsgemäß dadurch gelöst, dass ein erster Implantatbereich der mindestens drei Implantatbereiche durch einen ersten Endbereich des Implantats definiert wird, dass ein zweiter Implantatbereich der mindestens drei Implantatbereiche durch einen zweiten Endbereich des Implantats definiert wird und dass mindestens ein dritter Implantatbereich der mindestens drei Implantatbereiche zwischen dem ersten Implantatbereich und dem zweiten Implantatbereich angeordnet oder ausgebildet wird.

Die vorgeschlagene Weiterbildung des eingangs beschriebenen Verfahrens hat insbesondere die bereits oben im Zusammenhang mit bevorzugten Ausführungsformen medizinischer Implantat in Form medizinischer Clips beschriebenen Vorteile. Derart hergestellte medizinische Implantate können von Anwendern zuverlässig beispielsweise von einfarbig ausgebildeten Implantaten unterschieden werden. Farbig ausbilden ist insbesondere so zu verstehen, dass die mindestens drei Implantatbereiche durchgefärbt werden, also aus einem farbigen Werkstoff ausgebildet werden, oder dass nur die Implantatoberfläche eingefärbt wird, beispielsweise durch Beschichten, so dass ein farbiger Eindruck beim Betrachter entsteht. Zum Beispiel kann auch ein Werkstoff gewählt werden, um das Implantat auszubilden, welcher andersfarbig ist als eine Implantatoberfläche in den mindestens drei Implantatbereichen.

Vorteilhaft ist es, wenn mindestens zwei der mindestens drei Implantatbereiche gleichfarbig ausgebildet werden, wobei diese mindestens zwei Implantatbereiche nicht direkt aneinander angrenzen.

Günstig ist es, wenn mindestens zwei der mindestens drei Implantatbereiche gleichfarbig ausgebildet werden, wobei diese mindestens zwei Implantatbereiche nicht direkt aneinander angrenzen. Mit anderen Worten sind diese beiden Implantatbereiche voneinander beabstandet, beispielsweise sind sie durch einen weiteren, anders gefärbten Implantatbereich voneinander getrennt. Zwei Implantatbereiche gleichfarbig auszubilden hat insbesondere den Vorteil, dass das Implantat auch noch dann eindeutig identifizierbar ist, wenn einer der beiden gleichfarbig ausgebildeten Implantatbereiche nicht sichtbar ist, beispielsweise weil er durch ein Instrument oder Gewebe im Bereich des Operationssitus verdeckt ist. Eine eindeutige Trennung unterschiedlicher Implantatbereiche kann also durch entsprechende Einfärbung vorgegeben werden. Zudem lässt sich durch einen Anwender auch bei optisch ungünstigen Bedingungen wie insbesondere unter einem Mikroskop, bei einem minimal-invasiven chirurgischen Eingriff oder bei abgedunkelter Beleuchtung, eine Art des Clips sicher identifizieren, also beispielsweise temporäre oder permanente Clips.

Vorteilhaft ist es, wenn zwei aneinander angrenzende Implantatbereiche der mindestens drei Implantatbereiche einen farbigen Übergangsbereich der Oberfläche definieren und wenn der farbige Übergangsbereich an einem geometrisch definierten Bereich des Implantats angeordnet oder ausgebildet wird. Insbesondere kann der farbige Übergangsbereich in einem geometrisch definierten Bereich des Implantats angeordnet oder ausgebildet werden, dessen Querschnitt auf einer Bereichslänge, welche mindestens etwa 5%, insbesondere etwa 10%, einer Gesamtlänge des Implantats entspricht, konstant ist oder sich konisch verjüngt oder erweitert. So können insbesondere sauber getrennte Implantatbereiche unterschiedlicher Färbung erhalten werden, insbesondere in Bereichen, in denen sich ein Querschnitt des Implantats ändert oder konstant ist. Insbesondere bei bevorzugten Ausführungsformen des Verfahrens verwendete Schutzschichten lassen sich bei derart eindeutig definierten geometrischen Bereichen zuverlässig anbringen, um saubere scharfe Grenzen zwischen benachbarten Clipbereichen unterschiedlicher Farbe zu erhalten.

Um möglichst viele unterschiedliche Arten von Implantaten optisch voneinander unterscheiden zu können, ist es vorteilhaft, wenn mindestens einer der mindestens drei Implantatbereiche in einer der Farben Grün, Blau, Violett, Gelb oder goldfarbig gefärbt wird.

Günstig ist es, wenn ein erster Implantatbereich der mindestens drei Implantatbereiche durch einen ersten Endbereich des Implantats definiert wird, wenn ein zweiter Implantatbereich der mindestens drei Implantatbereiche durch einen zweiten Endbereich des Implantats definiert wird und wenn mindestens ein dritter Implantatbereich der mindestens drei Implantatbereiche zwischen dem ersten Implantatbereich und dem zweiten Implantatbereich angeordnet oder ausgebildet wird. So können insbesondere Implantate mit drei gefärbten Clipbereichen ausgebildet werden. Beispielsweise können zwei der drei Clipbereiche identisch farbig ausgebildet werden. Selbstverständlich kann das Implantat auch mit vier, fünf, sechs oder noch mehr unterschiedlich farbigen Clipbereichen ausgebildet werden. Denkbar ist insbesondere, benachbarte Clipbereiche abwechselnd unterschiedlich zu färben, also beispielsweise bei insgesamt sechs Clipbereichen nur zwei Farben zu deren Kennzeichnung vorzusehen. So können dann insbesondere auf einfache Weise gestreifte Implantate ausgebildet werden.

Um insbesondere eine gute Unterscheidbarkeit auch bei schlechten Lichtverhältnissen zu erreichen, ist es günstig, wenn der erste Implantatbereich und der zweite Implantatbereich heller gefärbt sind als der mindestens eine dritte Implantatbereich. So kann insbesondere erreicht werden, dass auch dann, wenn einer der beiden Implantatbereiche, welche sind der erste Implantatbereich und der zweite Implantatbereich, abgedeckt und für einen Anwender nicht sichtbar sind, doch der andere dieser beiden Implantatbereiche sichtbar bleibt und eine sichere Unterscheidung des mehrfarbigen Implantats von einem einfarbigen Implantat ermöglicht.

Vorteilhaft ist es, wenn entweder die Oberfläche des mindestens einen dritten Implantatbereichs gelb oder goldfarbig ausgebildet wird oder wenn die Oberflächen des ersten Implantatbereichs und des zweiten Implantatbereichs gelb oder goldfarbig ausgebildet werden. Beispielsweise kann eine gelbe oder goldfarbige Gestaltung der genannten Clipbereiche zur Kennzeichnung von temporären Implantaten genutzt werden, die lediglich temporär während eines chirurgischen Eingriffs genutzt werden, beispielsweise um eine optimale Form und Größe des endgültig zu implantierenden, permanenten Implantats zu ermitteln.

Vorteilhaft ist es, wenn die Oberfläche mindestens eines Implantatbereichs, insbesondere aller Implantatbereiche, der mindestens drei Implantatbereiche mit einer farbigen Beschichtung versehen wird. Farbige Beschichtungen lassen sich einfach und sicher an einem Implantat anbringen oder ausbilden. Insbesondere können diese auch dazu dienen, dass Implantate aus einem nicht körperverträglichen Werkstoff zur Ausbildung körperverträglicher Implantate genutzt werden können, da eine solche Beschichtung aus körperverträglichen Werkstoffen einen direkten Kontakt von Körpergewebe eines Patienten mit einem körperunverträglichen Werkstoff, aus dem das Implantat hergestellt ist, vermeiden hilft.

Vorzugsweise wird die Beschichtung mit einer Schichtdicke in einem Bereich von etwa 10 nm bis etwa 500 nm ausgebildet. Insbesondere kann die Schichtdicke einen Wert in einem Bereich von etwa 20 nm bis etwa 200 nm aufweisen. Beschichtungen mit einer Schichtdicke in den angegebenen Bereichen können insbesondere auch einen guten Korrosionsschutz für das Implantat selbst bieten. Dünne Beschichtungen können insbesondere kostengünstig ausgebildet werden. Zudem ist es möglich, beispielsweise Oxidschichten mit derartigen Schichtdicken auszubilden, um Interferenzfilter auf dem Implantat zu erhalten, die eine Farbwirkung der Oxidschicht vorgeben. Mit anderen Worten können unterschiedlich dicke Beschichtungen eine unterschiedliche Farbwirkung ergeben.

Auf einfache Weise kann die Beschichtung in Form einer Oxidschicht durch anodische Oxidation ausgebildet werden. Bei der anodischen Oxidation wird eine Schicht aus einem Oxid des Werkstoffs ausgebildet, aus dem das Implantat hergestellt ist. Beispielsweise kann die Oxidschicht aus Titandioxid ausgebildet werden, wenn das Implantat aus Titan ausgebildet ist.

Die Herstellung medizinischer Implantate lässt sich vereinfachen, wenn Implantatbereiche mit gleichfarbig ausgebildeter Oberfläche mit einer identischen Beschichtung versehen werden. So können zwei oder mehr Implantatbereiche in einem Verfahrensschritt mit einer gleichfarbig ausgebildeten Oberfläche ausgestattet werden.

Vorzugsweise wird die Beschichtung durch Vergolden ausgebildet. Beispielsweise kann dies mittels eines Tauchverfahrens erfolgen. Eine derartige Beschichtung hat insbesondere den Vorteil, dass sie auch bei Werkstoffen möglich ist, die für eine anodische Oxidation zur Ausbildung einer Oxidschicht ungeeignet sind. Beispielsweise können Beschichtungen durch Vergolden bei Implantaten genutzt werden, die aus einem Kobalt-Chrom-Stahl ausgebildet sind oder aus einem anderen Werkstoff, welcher Kobalt und/oder Chrom enthält.

Günstigerweise wird mindestens ein Implantatbereich der mindestens drei Implantatbereiche unbeschichtet belassen. Dies kann insbesondere dann vorgesehen werden, wenn der Implantatwerkstoff körperverträglich ist oder bereits eine Passivierungsschicht aufweist. So kann mindestens ein Verfahrensschritt eingespart werden, nämlich der, um den mindestens einen Implantatbereich zum Einfärben zu beschichten.

Gemäß einer weiteren bevorzugten Ausführungsform der Erfindung kann vorgesehen sein, dass das Implantat aus einem körperverträglichen Metall und/oder einem anodisch oxidierbaren Metall oder einer metallischen Legierung ausgebildet wird. Insbesondere kann als Metall Titan, Aluminium oder Tantal eingesetzt werden, welches vom Implantatwerkstoff umfasst ist. Bei derartigen Implantaten können Abstoßungsreaktionen durch den Körper eines Patienten nach Implantieren des Implantats minimiert werden.

Vorteilhaft ist es, wenn das Implantat in einen Elektrolyt angebracht wird und wenn zum anodischen Oxidieren das Implantat mit der Anode einer Gleichspannungsquelle verbunden und mit einer Anodisierungsspannung beaufschlagt wird. Beim Elektrolyt kann es sich insbesondere um eine verdünnte Säure handeln. Bei dem beschriebenen Vorgehen kann auf einfache Weise eine Oxidschicht ausgebildet werden, und zwar eine zum Werkstoff, aus dem das Implantat ausgebildet ist, korrespondierende Oxidschicht.

Auf einfache Weise lässt sich das Implantat mit charakteristischen Farben einfärben, wenn zur Ausbildung unterschiedlicher Farben beim anodischen Oxidieren unterschiedliche Anodisierungsspannungen an das Implantat angelegt werden. Beispielsweise können so unterschiedliche Implantatbereiche unterschiedlich eingefärbt werden, indem einer oder mehrere Implantatbereiche vor dem anodischen Oxidieren teilweise abgedeckt werden und dann das Implantat mit einer ersten Anodisierungsspannung beaufschlagt wird, um auf dem nicht abgedeckten Implantatbereich eine erste Oxidschicht auszubilden. In einem weiteren Verfahrensschritt können dann die bereits mit einer Oxidschicht beschichteten Clipbereiche abgedeckt werden und das Implantat mit einer anderen, beispielsweise niedrigeren, Anodisierungsspannung beaufschlagt werden, um die verbleibenden Implantatbereiche mit einer andersfarbigen Oxidschicht zu versehen.

Gemäß einer bevorzugten Ausführungsform des Verfahrens kann vorgesehen sein, dass mindestens einer der mindestens drei Implantatbereiche mit einer Schutzschicht bedeckt wird, dass dann das Implantat mit einer ersten Anodisierungsspannung anodisch oxidiert wird zur Ausbildung einer ersten Oxidschicht auf der Oberfläche des Implantats, die nicht mit der Schutzschicht bedeckt ist, dass die Schutzschicht entfernt wird, dass dann das Implantat mit einer zweiten Anodisierungsspannung anodisch oxidiert wird zur Ausbildung einer zweiten Oxidschicht auf der Oberfläche des Implantats, wobei die erste Anodisierungsspannung größer ist als die zweite Anodisierungsspannung. Diese Vorgehensweise hat insbesondere den Vorteil, dass die zuerst ausgebildete Oxidschicht durch die im zweiten Anodisierungsschritt ausgebildete zweite Oxidschicht nicht verändert wird, da die zweite Anodisierungsspannung geringer ist als die erste Anodisierungsspannung. So ändert sich die erste Oxidschicht nicht. Dagegen werden der zweite oder auch noch weitere Implantatbereiche, die zunächst mit einer Schutzschicht bedeckt waren, mit der zweiten Oxidschicht versehen, die aufgrund der anderen Anodisierungsspannung eine andere Farbwirkung ergibt.

Auf einfache Weise lässt sich das Implantat teilweise bedecken, wenn die Schutzschicht durch eine Harzschicht ausgebildet wird. Beispielsweise kann das Implantat in Harz eingetaucht werden. So lässt sich eine Tauchbeschichtung erzielen. Ist das Harz getrocknet und ausgehärtet, können auch noch weitere Bereiche des Implantats optional mit einer solchen Schutzschicht bedeckt werden.

Gemäß der Erfindung ist das Implantat in Form eines medizinischen Clips ausgebildet. Bei einer nicht erfindungsgemäßen Ausgestaltung wird das Implantat in Form einer medizinischen Schraube ausgebildet. Insbesondere kann die medizinische Schraube in Form einer Knochenschraube oder einer Pedikelschraube ausgebildet werden. Selbstverständlich ist die nicht erfindungsgemäße Anwendung des beschriebenen Verfahrens nicht auf medizinische Implantate in Form von medizinischen Schrauben limitiert. Grundsätzlich kann das medizinische Implantat jede Art von Implantat sein.

Vorteilhaft ist es, wenn der medizinische Clip mit zwei Klemmarmen und einem vorspannenden Element ausgebildet wird, wenn jeweils ein Klemmarm an einem freien Ende des vorspannenden Elements angeordnet oder ausgebildet wird und wenn die zwei Klemmarme in einer Grundstellung aneinander anliegen und entgegen der Wirkung des vorspannenden Elements in eine Öffnungsstellung voneinander weg bewegbar sind. Beispielsweise können die drei Implantatbereiche beim medizinischen Clip einerseits durch freie Ende der zwei Klemmarme und andererseits durch das vorspannende Element sowie einen Bereich zwischen diesen gebildet werden. Ein solcher medizinischer Clip lässt sich insbesondere als Aneurysmenclip zur Behandlung eines Aneurysmas einsetzen.

Um eine optimale Einfärbung des Clips zu erreichen, also insbesondere auch von Flächen, die in der Grundstellung aneinander anliegen, beispielsweise Klemmflächen der zwei Klemmarme des Clips, ist es günstig, wenn vor dem Abdecken eines der mindestens drei Implantatbereiche mit der Schutzschicht der Clip geöffnet wird. So kann beispielsweise im Bereich der Klemmarme jeder Klemmarm von allen Seiten gleichmäßig mit einer Schutzschicht abgedeckt und dann wie oben beschrieben in einem zweiten Anodisierungsschritt mit einer Oxidschicht versehen werden.

Ferner wird die Verwendung eines der oben beschriebenen Verfahren zum Herstellen eines medizinischen Implantats vorgeschlagen, insbesondere zum Herstellen einer medizinischen Schraube oder eines der oben beschriebenen medizinischen Clips, wobei nur das Herstellen eines medizinischen Clips unter die beanspruchte Erfindung fällt.

Auf diese Weise lassen sich medizinische Implantate, insbesondere medizinische Clips, in gewünschter Weise für einen Anwender sicher unterscheidbar einfärben.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines Ausführungsbeispiels eines Applikationsinstruments mit einem Ausführungsbeispiel eines vom Applikationsinstrument gehaltenen medizinischen Implantats in Form eines temporären Implantats;
- Figur 2:: eine Draufsicht auf ein Ausführungsbeispiel eines temporären Implantats;
- Figur 3:: eine Ansicht des Implantats aus Figur 2 in Richtung des Pfeils A;
- Figur 4:: eine Draufsicht auf ein weiteres Ausführungsbeispiel eines temporären Implantats;
- Figur 5:: eine Ansicht des Clips aus Figur 4 in Richtung des Pfeils B;
- Figur 6:: eine Draufsicht auf ein weiteres Ausführungsbeispiel eines temporären Implantats;
- Figur 7:: eine Ansicht des Implantats aus Figur 6 in Richtung des Pfeils C;
- Figur 8:: eine Draufsicht auf ein weiteres Ausführungsbeispiel eines temporären Implantats;
- Figur 9:: eine Ansicht des Implantats aus Figur 8 in Richtung des Pfeils D;
- Figur 10:: eine Draufsicht auf ein weiteres Ausführungsbeispiel eines temporären Implantats;
- Figur 11:: eine Ansicht des Implantats aus Figur 10 in Richtung des Pfeils E;
- Figur 12:: eine Draufsicht auf ein weiteres Ausführungsbeispiel eines temporären Implantats;
- Figur 13:: eine Draufsicht auf ein Ausführungsbeispiel eines permanenten Implantats, welches zu dem in Figur 2 dargestellten temporären Implantat korrespondiert;
- Figur 14:: eine Ansicht des Implantats aus Figur 13 in Richtung des Pfeils F;
- Figur 15:: eine Draufsicht auf ein Ausführungsbeispiel eines permanenten Implantats, welches zu dem in Figur 4 dargestellten temporären Implantat korrespondiert;
- Figur 16:: eine Ansicht des Implantats aus Figur 15 in Richtung des Pfeils G;
- Figur 17:: eine Draufsicht auf ein Ausführungsbeispiel eines permanenten Implantats, welches zu dem in Figur 6 dargestellten temporären Implantat korrespondiert;
- Figur 18:: eine Ansicht des Implantats aus Figur 17 in Richtung des Pfeils H;
- Figur 19:: eine Draufsicht auf ein Ausführungsbeispiel eines permanenten Implantats, welches zu dem in Figur 8 dargestellten temporären Implantat korrespondiert;
- Figur 20:: eine Ansicht des Implantats aus Figur 19 in Richtung des Pfeils I;
- Figur 21:: eine Draufsicht auf ein Ausführungsbeispiel eines permanenten Implantats, welches zu dem in Figur 10 dargestellten temporären Implantat korrespondiert;
- Figur 22:: eine Ansicht des Implantats aus Figur 21 in Richtung des Pfeils K;
- Figur 23:: eine Draufsicht auf ein Ausführungsbeispiel eines permanenten Implantats, welches zu dem in Figur 12 dargestellten temporären Implantat korrespondiert;
- Figur 24:: eine schematische Darstellung eines Implantats vor dem anodischen Oxidieren, welches zwei mit einer Schutzschicht versehene Implantatbereiche aufweist;
- Figur 25:: eine schematische Ansicht des Implantats aus Figur 24 mit einem Implantatbereich, welcher bei der anodischen Oxidation nicht mit einer Schutzschicht bedeckt war und auf dessen Oberfläche eine Oxidschicht ausgebildet ist;
- Figur 26:: eine schematische Ansicht des Implantats aus Figur 25 mit entfernter Schutzschicht;
- Figur 27:: eine schematische Darstellung des Implantats aus Figur 25 nach Ausbilden einer Oxidschicht an den Implantatbereichen, die wie in Figur 24 dargestellt vor dem ersten anodischen Oxidationsschritt mit einer Schutzschicht bedeckt waren;
- Figur 28:: eine schematische Darstellung der Funktionsweise des Interferenzeffekts einer dünnen Oxidschicht; und
- Figur 29:: ein schematischer Aufbau einer Anordnung zum Ausbilden einer anodischen Oxidschicht auf einem Implantat.

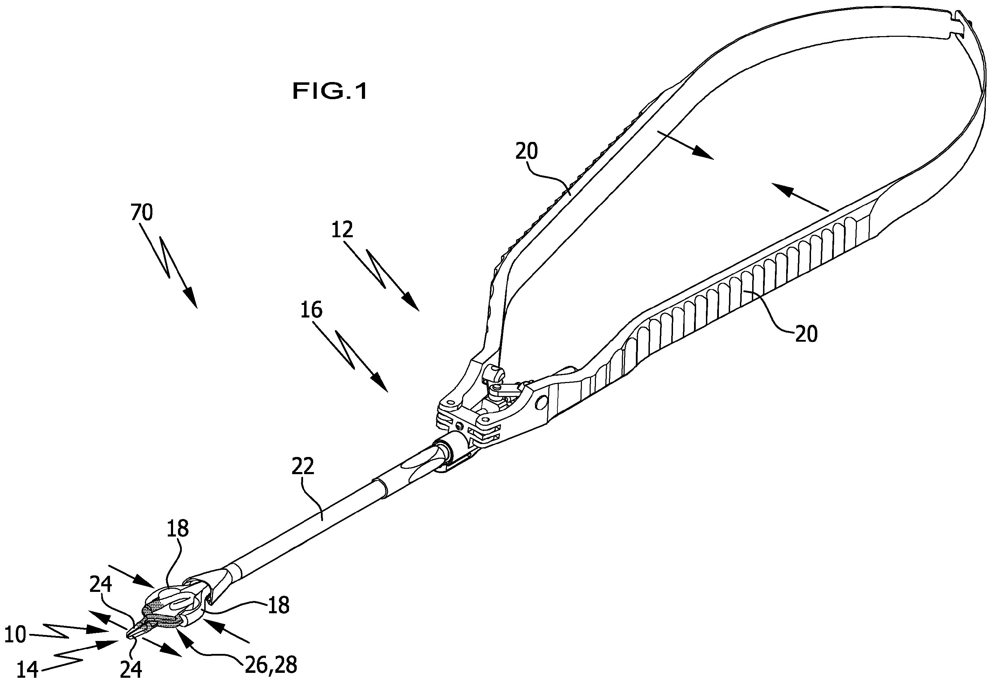

In Figur 1 ist beispielhaft ein Ausführungsbeispiel eines medizinischen Implantats 10 zusammen mit einem Ausführungsbeispiel eines Applikationsinstruments 12 dargestellt.

Das Implantat 10 ist in Form eines medizinischen Clips 14 ausgebildet, das Applikationsinstrument 12 in Form einer Clipanlegezange 16.

Die Clipanlegezange 16 ist in Form eines Schiebeschaftinstruments ausgebildet und umfasst zwei ein distales Ende der Clipanlegezange 16 bildende Werkzeugelemente 18, zwischen denen ein Clip 14 aufgenommen werden kann. Gegeneinander verschwenkbare Branchen 20 am proximalen Ende der Clipanlegezange 16 ermöglichen eine Bewegung eines Schafts 22 in distaler Richtung auf die Werkzeugelemente 18 hin, um diese aufeinander zu zu bewegen. Bei dieser Bewegung wird der chirurgische Clip geöffnet, das heißt zwei in einer Grundstellung, die schematisch in Figur 1 dargestellt ist, aneinander anliegende Endbereiche von Klemmarmen 24 des Clips 14 werden dann auseinander bewegt.

Der in Figur 1 dargestellte medizinische Clip 14 ist in Form eines Aneurysmenclips 26 ausgebildet zur Behandlung von Aneurysmen. Es handelt sich bei dem in Figur 1 schematisch dargestellten Aneurysmenclip 26 um einen sogenannten temporären Clip 28. Dieser ist dazu vorgesehen, bei einem chirurgischen Eingriff ein Aneurysma temporär abzuklemmen. Ist Form und Größe des dauerhaft zu implantierenden permanenten Clips 30 festgestellt, kann ein Operateur den temporären Clip 28 entfernen und durch den entsprechenden permanenten Clip 30, welcher dauerhaft im Körper eines Patienten verbleiben kann, ersetzen.

Um temporäre Clips 28 und permanente Clips 30 voneinander unterscheiden zu können, sind diese unterschiedlich gefärbt.

Der Clip 14 umfasst ein vorspannendes Element 32 in Form einer Schraubenfeder mit 1,5 Windungen, deren freie Enden mit den Klemmarmen 24 verbunden sind.

Zwischen dem vorspannenden Element 32 und den Klemmarmen 24 ist ein Schlussbereich 34 ausgebildet, in welchem sich die beiden Klemmarme 24 einerseits und die freien Enden der vorspannenden Elemente 32 verbindende Clipabschnitte 36 kreuzen.

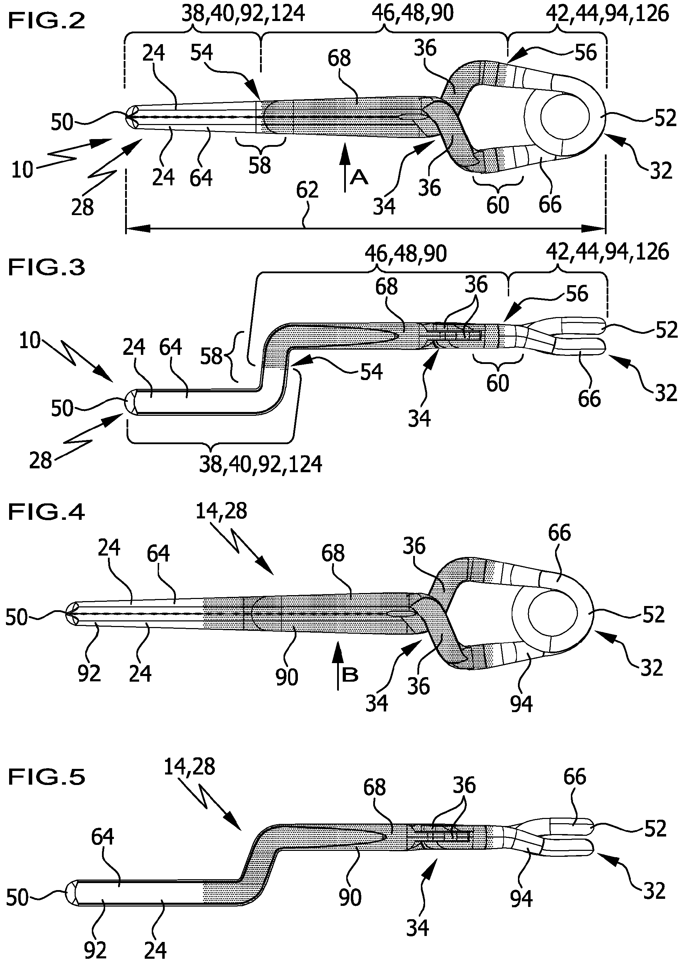

Das in Figur 2 schematisch dargestellte Implantat 14 definiert einen ersten Implantatbereich 38, welcher einen ersten Clipbereich 40 definiert, einen zweiten Implantatbereich 42, welcher einen zweiten Clipbereich definiert und einen dritten Implantatbereich 46, welcher einen dritten Clipbereich 48 definiert.

Der erste Clipbereich 40, welcher einen ersten Endbereich 124 definiert, erstreckt sich ausgehend von einem distalen Ende 50 der Klemmarme 24 in Richtung auf ein proximales Ende 52 des Implantats 10 hin. Der zweite Clipbereich 44, welcher einen zweiten Endbereich 126 definiert, erstreckt sich ausgehend vom proximalen Ende 52 in distaler Richtung. Der dritte Clipbereich 48 erstreckt sich zwischen dem ersten Clipbereich 40 und dem zweiten Clipbereich 44.

In Figur 2 ist der Clip 14 in einer Grundstellung dargestellt, in welcher die Klemmarme 24 aneinander anliegen. Sie können entgegen der Wirkung des vorspannenden Elements 32 in eine Öffnungsstellung voneinander weg bewegt werden.

Die Clipbereiche 40, 44 und 48 sind farbig ausgebildet. Mit anderen Worten bilden die genannten Clipbereiche 40, 44 und 48 jeweils einen Teil einer Oberfläche des Clips 14 aus und sind unterschiedlich gefärbt.

Bei dem in Figur 2 dargestellten Ausführungsbeispiel sind die Implantatbereiche 38 und 42 heller gefärbt als der dritte Implantatbereich 48.

Zwischen dem ersten Implantatbereich 38 und dem dritten Implantatbereich 46 ist ein erster Übergangsbereich 54 definiert, zwischen dem zweiten Implantatbereich 42 und dem dritten Implantatbereich 46 ein zweiter Übergangsbereich 56. Die Übergangsbereiche 54 und 56 sind jeweils an einem ersten geometrischen definierten Bereich 58 beziehungsweise einem zweiten geometrischen definierten Bereich 60 ausgebildet. Die geometrischen definierten Bereiche 58 und 60 des Clips 14 sind dadurch gekennzeichnet, dass ein Querschnitt des Clips 14 auf einer Bereichslänge, welche mindestens etwa 5% einer Gesamtlänge 62 des Clips 14 entspricht, konstant ist oder sich ändert. Der geometrisch definierte Bereich 58 an den Klemmarmen 24 zeichnet sich durch einen konstanten Querschnitt aus. Der geometrisch definierte Bereich 60, welcher sich direkt an das vorspannende Element 32 anschließt, weist einen sich vom vorspannenden Element 32 in Richtung auf den Schlussbereich 34 hin konische erweiternden Querschnitt auf, welcher in einen zylindrischen Abschnitt des Clips 10 übergeht.

Die drei Implantatbereiche 38, 42 und 46 sind jeweils mit einer Beschichtung 64, 66 und 68 versehen. Die Herstellung dieser Beschichtungen 64, 66 und 68 sowie deren Aufbau werden nachfolgend noch näher erläutert.

Die Figuren 4 bis 12 zeigen unterschiedliche Ausführungsbeispiele von chirurgischen Clips 14. Diese unterscheiden sich ausschließlich durch eine Form ihrer Klemmarme 24. Daher sind alle Ausführungsbeispiele mit denselben Bezugszeichen versehen.

Das Ausführungsbeispiel der Figuren 2 und 3 zeigt doppelt abgewinkelte Klemmarme 24. Ein weiteres Ausführungsbeispiel eines Clips 14 mit doppelt abgewinkelten Klemmarmen, die im Vergleich zum Ausführungsbeispiel der Figuren 2 und 3 jedoch etwas länger sind, ist in den Figuren 4 und 5 schematisch dargestellt.

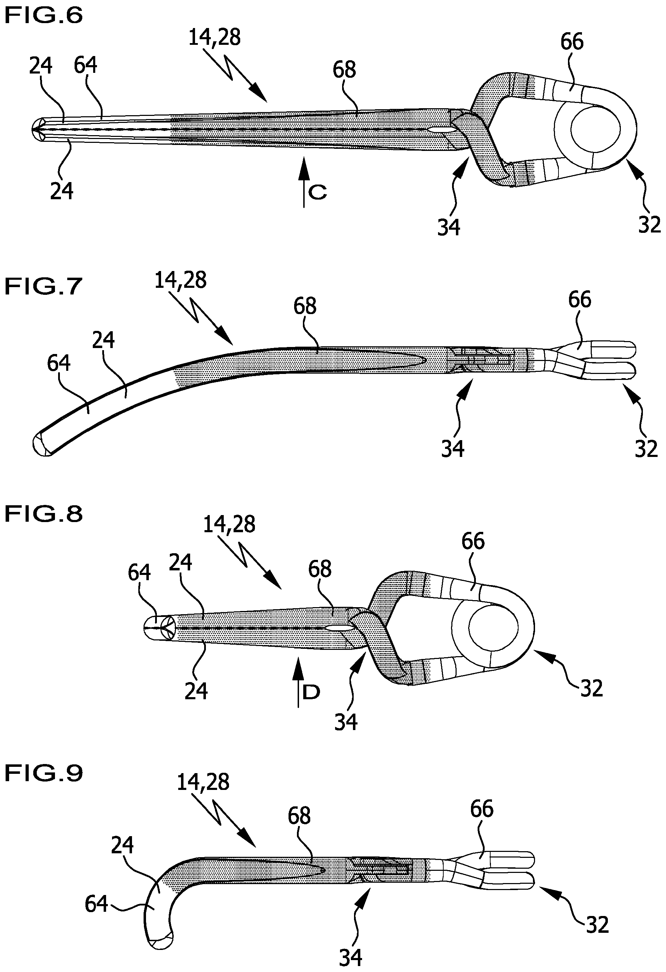

Die Klemmarme 24 bei dem Ausführungsbeispiel des Clips 14 aus den Figuren 6 und 7 sind lang gezogen gekrümmt mit einem relativ großen Krümmungsradius.

Beim Ausführungsbeispiel des Clips 14 der Figuren 8 und 9 sind die Klemmarme 24 geschwungen gekrümmt, jedoch mit einem im Vergleich zum Ausführungsbeispiel der Figuren 6 und 7 deutlich geringeren Krümmungsradius.

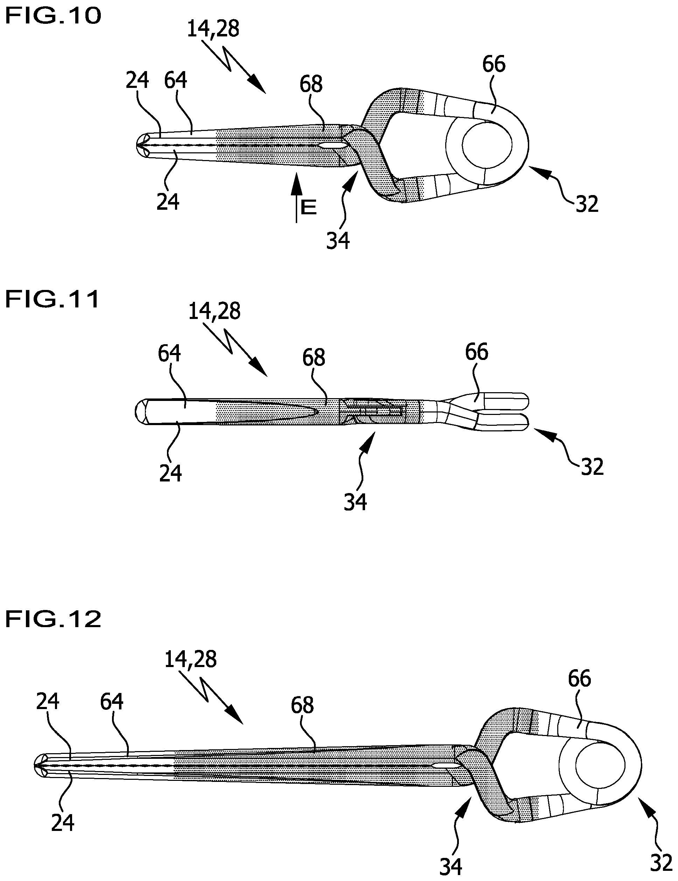

Das Ausführungsbeispiel des Clips der Figuren 10 und 11 zeigt geradlinig verlaufende Klemmarme 24.

Die Klemmarme des in Figur 12 dargestellten Ausführungsbeispiels des Clips 14 verlaufen ebenfalls geradlinig, sind jedoch im Vergleich zum Ausführungsbeispiel der Figuren 10 und 11 deutlich länger.

Die Figuren 1 bis 12 zeigen temporäre Clips 28.

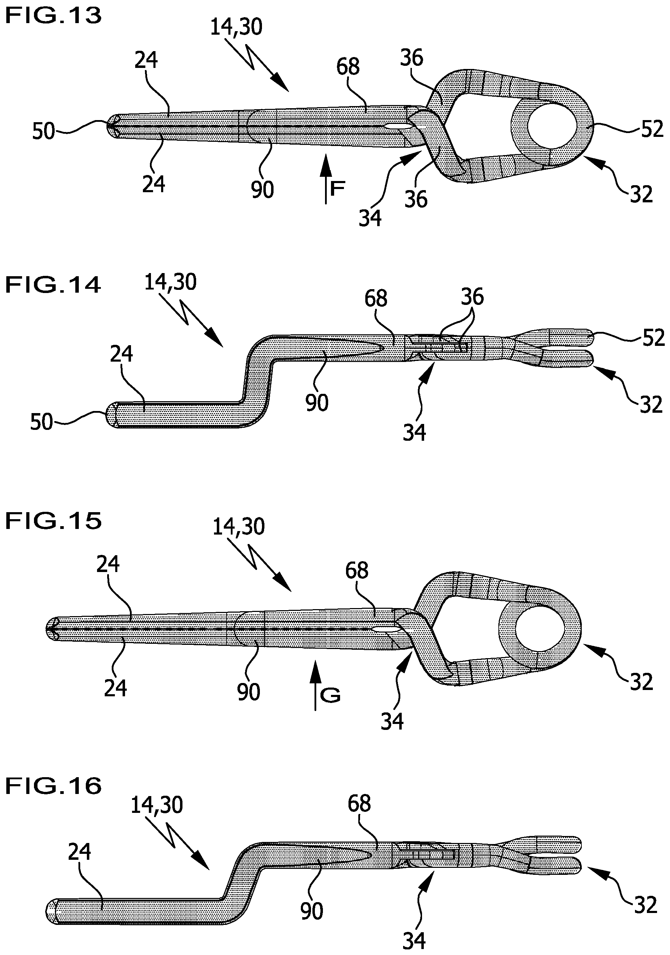

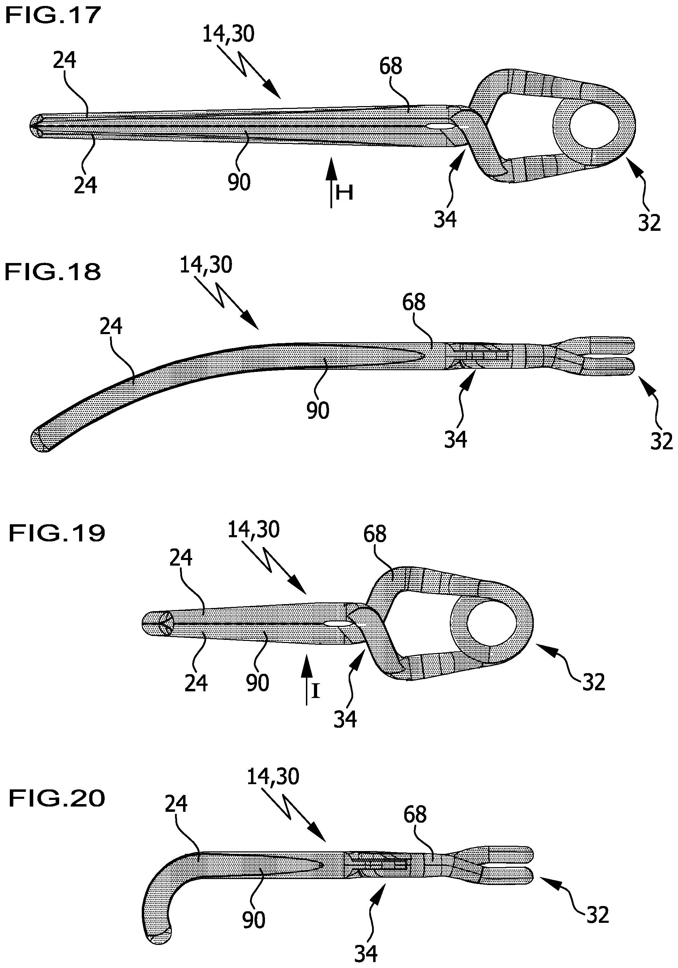

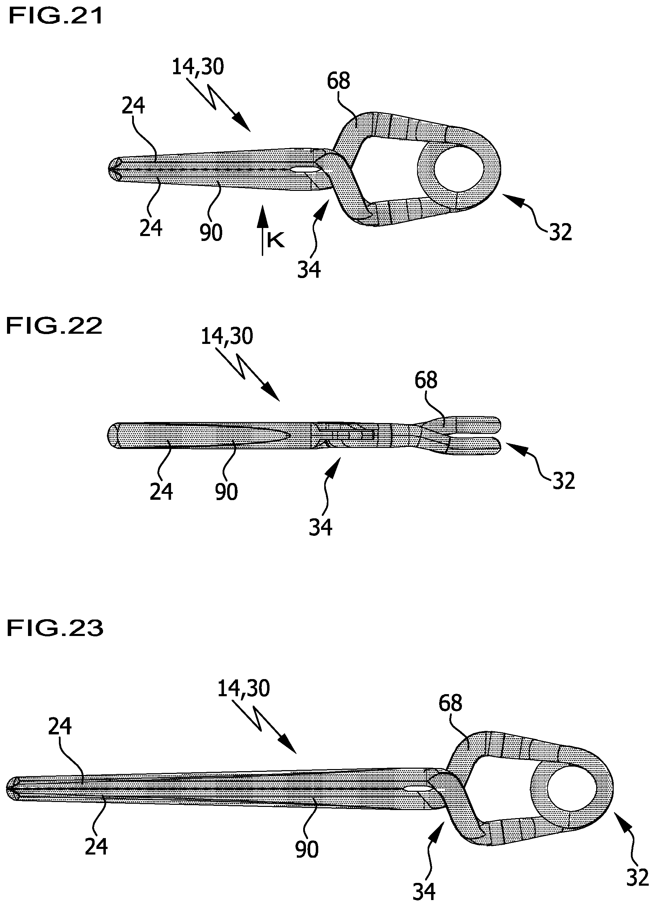

Die in den Figuren 13 bis 23 dargestellten Ausführungsbeispiele permanenter Clips 30 stimmen in Form und Größe mit den jeweils zugeordneten Ausführungsbeispielen der temporären Clips 28 der Figuren 2 bis 12 überein. Sie unterscheiden sich jedoch von den temporären Clips 28 durch ihre Farbgebung. Alle Ausführungsbeispiele der permanenten Clips, die in den Figuren 13 bis 23 schematisch dargestellt sind, sind einfarbig ausgebildet. Sie sind in einer Farbe eingefärbt, die der Farbgebung des dritten Implantatbereichs 46 der temporären Clips 28 entspricht. Dadurch ist eine einfache Zuordnung temporärer Clips 28 und entsprechender permanenter Clips 30 möglich.

Die sich in Form und Größe unterscheidenden Ausführungsbeispiele permanenter Clips der Figuren 13 und 14, 15 und 16, 17 und 18, 19 und 20, 21 und 22 sowie 23 unterscheiden sich optional in ihrer Farbgebung. Beispielsweise kann ein Ausführungsbeispiel grün sein, ein anderes Ausführungsbeispiel blau, ein weiteres Ausführungsbeispiel gelb, ein weiteres Ausführungsbeispiel rot und weiteres Ausführungsbeispiel violett. Entsprechend sind dann die dritten Implantatbereiche 16 beziehungsweise die dritten Clipbereiche 48 der temporären Clips 28 entsprechend den permanenten Clips 30 identischer Bauform und Größe eingefärbt.

Um die temporären Clips 28 von den permanenten Clips 30 sicher unterscheiden zu können, sind bei den temporären Clips 28 die ersten Clipbereiche 40 und die zweiten Clipbereiche 44 anders eingefärbt als die dritten Clipbereiche 48.

Bei den in den Figuren 1 bis 12 dargestellten Ausführungsbeispielen sind die ersten und zweiten Clipbereiche 40, 44 gelb oder goldfarbig gefärbt. Sie sind damit deutlich heller als die dritten Implantatbereiche 46 beziehungsweise die dritten Clipbereiche 48 eingefärbt. Ein Anwender kann dann die temporären Clips 28 auch dann noch sicher von permanenten Clips 30 unterscheiden, wenn die temporären Clips 28 beispielsweise mit der in Figur 1 schematisch dargestellten Clipanlegezange 16 gehalten sind und die vorspannenden Elemente 32 dabei nur schlecht oder gar nicht erkennbar sind.

Die ersten Implantatbereiche 38 der temporären Clips 28 sind jedoch gut erkennbar, insbesondere auch unter einem Mikroskop, so dass ein Anwender sofort erkennt, insbesondere auch bei einem minimalinvasiven chirurgischen Eingriff, ob er einen temporären Clip 28 handhabt und einen permanenten Clip 30.

Sowohl die permanenten Clips 30 als auch die Clipbereiche 40, 44 und 48 der beschriebenen und in den Figuren dargestellten Ausführungsbeispiele temporärer Clips 28 sind bei den dargestellten Ausführungsbeispielen durch anodische Oxidation ausgebildet.

Dazu wird zunächst der jeweilige Clip 28 beziehungsweise 30 aus einem körperverträglichen Metall, bei den Ausführungsbeispielen wird hierfür Titan, Aluminium oder Tantal eingesetzt, in der gewünschten Form ausgebildet, die beispielhaft in den Figuren 2 bis 23 dargestellt ist.

Bei alternativen Ausführungsbeispielen ist der jeweilige Clip 28, 30 aus einer metallischen Legierung ausgebildet, die Kobalt und/oder Chrom und/oder Nickel enthält.

Bei einem Ausführungsbeispiel sind die Clips aus Phynox ausgebildet.

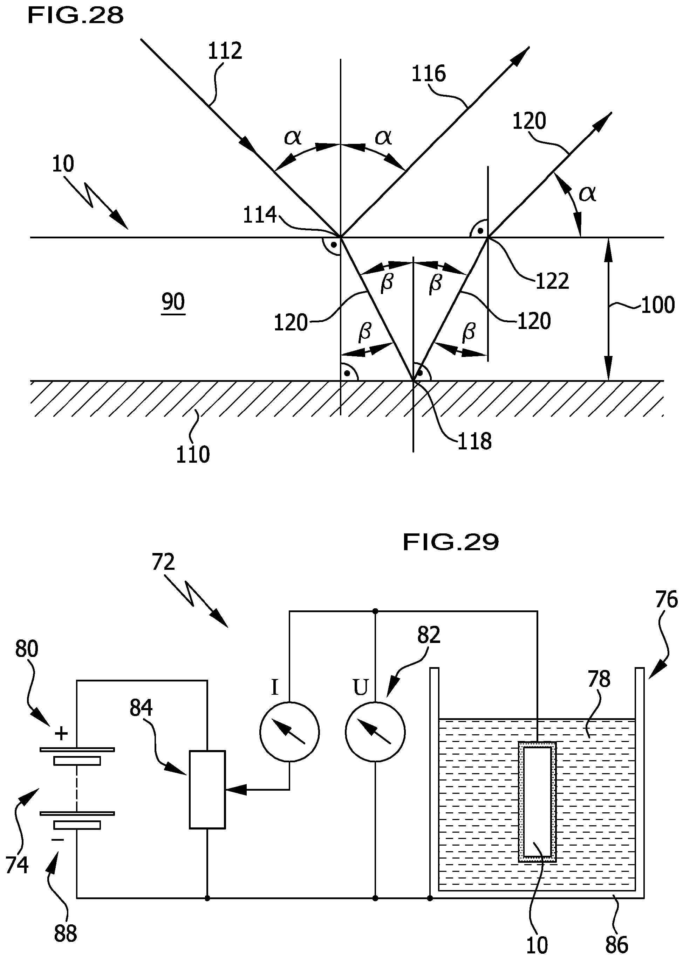

Bei Werkstoffen, welche anodisch oxidierbar sind, wie beispielsweise Titan, Aluminium und Tantal, kann eine wie schematisch in Figur 29 dargestellte Beschichtungseinrichtung 72 eingesetzt werden. Die Beschichtungseinrichtung 72 umfasst eine Gleichspannungsquelle 74 sowie eine Elektrolysezelle 76, die mit einem Elektrolyt 78 in Form einer verdünnten Säure befüllt ist.

Das zu beschichtende Implantat 10 wird in den Elektrolyt 78 eingebracht, so dass dieses vollständig vom Elektrolyt 78 umgeben ist.

Das Implantat 10 wird mit dem Pluspol 80, also der Anode, der Gleichspannungsquelle 74 verbunden und eine Anodisierspannung 82 mittels eines Potentiometers 84 eingestellt. Als Gegenelektrode dient bei dem in Figur 29 dargestellten Ausführungsbeispiel der Beschichtungseinrichtung 72 ein den Elektrolyt 78 aufnehmender Behälter 86, welcher elektrisch leitend mit dem Minuspol 88, also der Kathode, der Gleichspannungsquelle 74 verbunden ist.

Mit der Beschichtungseinrichtung 72 werden die in den Figuren 13 bis 23 dargestellten Ausführungsbeispiele permanenter Clips 30 in einem einzigen Anodisierschritt mit einer Oxidschicht versehen. Hierfür wird je nach gewünschter Farbwirkung die Anodisierspannung 82 mit dem Potentiometer 84 vorgegeben. Die permanenten Clips werden wie beschrieben in den Elektrolyt 78 vollständig eingetaucht und mit der Anode der Gleichspannungsquelle 74 elektrisch leitend verbunden. Abhängig von der Anodisierspannung 82 werden dann die Oxidschichten in einer gewünschten Farbe, beispielsweise einer beliebigen Spektralfarbe wie insbesondere grün, blau, violett, rot oder gelb, eingefärbt.

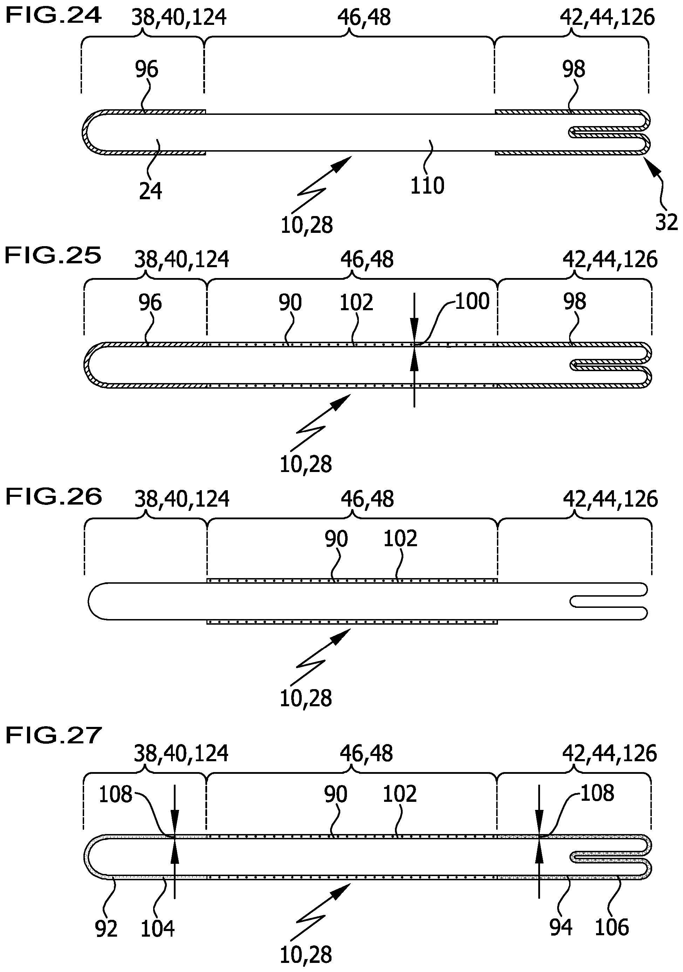

Zum Ausbilden einer farbigen Beschichtung 90 der Implantatbereiche 38, 42 und 46 der in den Figuren 2 bis 12 dargestellten Ausführungsbeispiele temporärer Clips 28 wird wie nachfolgend in Verbindung mit den Figuren 24 bis 27 näher erläutert vorgegangen.

Um in einem ersten Schritt den dritten Implantatbereich 46 mit der Beschichtung 90 zu versehen, werden zunächst die ersten und zweiten Implantatbereiche 38 und 40 mit einer Schutzschicht 96 beziehungsweise 98 versehen, die die jeweiligen Implantatbereiche 38, 42 vollständig bedeckt. Unbeschichtet bleibt dann lediglich der dritte Implantatbereich 46.

Wird das so mit den Schutzschichten 96 und 98 teilweise abgedeckte Implantat 10 in die Elektrolysezelle 76 in der oben beschriebenen Weise eingebracht, kann die Beschichtung 90 werden durch Anlegen einer ersten Anodisierspannung U₁ ausgebildet.

Die Beschichtung wird mit einer Dicke 100 ausgebildet. Sie bedeckt den dritten Implantatbereich 46 vollständig. Eine Ausbildung einer solchen Beschichtung 90 in Form einer solchen Oxidschicht 102 ist in den zweiten und dritten Implantatbereichen 42, 46 nicht möglich, da sie durch die Schutzschichten 96 und 98 bedeckt und dadurch passiviert sind.

Um auch die Implantatbereiche 42 und 46 mit einer Beschichtung 92 beziehungsweise 94 zu versehen, werden zunächst die Schutzschichten 96 und 98 entfernt. Das so vorbereitete Implantat 10 ist schematisch in Figur 26 abgebildet. Lediglich der dritte Implantatbereich 46 ist mit der Beschichtung 90 versehen.

Wird nun das so vorbereitete Implantat 10 wiederum in die Elektrolysezelle 76 eingebracht und mit dem Pluspol 80 der Gleichspannungsquelle 74 verbunden und eine zweite Anodisierspannung U₂ angelegt, die kleiner ist als die erste Anodisierspannung U₁, bildet sich auch an den Implantatbereichen 38 und 42 jeweils eine Oxidschicht 104 und 106 aus. Aufgrund der geringeren Anodisierspannung U₂ wird die bereits ausgebildete Oxidschicht 102 durch diesen zweiten Oxidationsschritt nicht weiter verändert.

Die Oxidschichten 104 und 106 weisen jeweils eine Dicke 108 auf.

Alle in den Figuren 2 bis 12 dargestellten und oben beschriebenen Ausführungsbeispiele temporärer Clips 28 können in der beschriebenen Weise mit drei unterschiedlich gefärbten Clipbereichen 40, 44 und 48 ausgebildet werden. Wie bereits erwähnt erfolgt eine Zuordnung zu einander korrespondierender permanenter Clips 30 und temporärer Clips 28 durch die identische Ausbildung mindestens eines der drei Implantatbereiche 38, 42 und 46 mit einer Beschichtung 90, die einer Beschichtung 90 der permanenten Clips 30 entspricht.

Die Beschichtungen 90, 92 und 94 weisen eine Dicke 100 beziehungsweise 108 auf, die in einem Bereich von etwa 10 nm bis etwa 500 nm liegt. Bei Ausführungsbeispielen liegt sie in einem Bereich von etwa 20 nm bis etwa 200 nm.

Die Farbwirkung der wie beschrieben ausgebildeten Oxidschichten 102 bis 106 wird nachfolgend in Verbindung mit Figur 28 erläutert.

In Figur 28 ist schematisch die Oxidschicht 90 dargestellt, die auf dem anodisch oxidierbaren Werkstoff 110, aus dem das Implantats 10 ausgebildet ist, ausgebildet ist.

Einfallendes Licht 112 wird am Auftreffpunkt 114 auf der Oxidschicht 90 teilweise reflektiert und mit einem Ausfallswinkel α, welcher einem Einfallswinkel α des Lichts 112 auf die Oxidschicht entspricht, als Wellenfront 116 abgelenkt.

Ein Teil des Lichts 112 dringt in die Beschichtung 90 ein und wird in diesem optisch dichteren Medium zum Lot hin gebrochen. Am Punkt 118, also an der Grenzfläche zwischen der Beschichtung 90 und dem Werkstoff 110, erfolgt eine Totalreflexion der Wellenfront 120 am optisch dichteren Medium, welche dann am Austrittspunkt 122 wieder aus der Beschichtung 90 austritt. Die beiden Wellenfronten 116 und 120 überlagern sich interferometrisch und ergeben in Abhängigkeit der Art und Dicke 100 der Beschichtung 90 eine unterschiedliche Farbwirkung der Beschichtung 90.

Werden die Implantate 10 nicht aus einem oxidierbaren Material ausgebildet, können die Beschichtungen auch in Tauchverfahren realisiert werden. Beispielsweise werden die ersten und zweiten Implantatbereiche 38 und 42 bei Ausführungsbeispielen der Implantate 10 durch Tauchbeschichtung mit einer Goldbeschichtung versehen. Die dritten Implantatbereiche 46 werden entweder nicht beschichtet oder vor der Beschichtung der ersten und zweiten Implantatbereiche 38 und 42 mit einer anderen Beschichtung versehen, die sich farblich von einer goldenen Beschichtung unterscheidet. In diesem Fall sind dann die permanenten Clips 30, die in den Figuren 13 bis 23 schematisch dargestellt sind, aus demselben Werkstoff wie die temporären Clips 28 ausgebildet und wiederum mit einer Beschichtung 90 versehen, die der Beschichtung 90 der dritten Implantatbereiche 46 der temporären Clips 28 entspricht.

Bei weiteren Ausführungsbeispielen ist das in Verbindung mit den Figuren 24 bis 27 beschriebene Beschichtungsverfahren umgekehrt realisiert. In diesem Fall wurde zuerst der dritte Implantatbereich 46 mit der Schutzschicht 96 beziehungsweise 98 bedeckt und die ersten und zweiten Implantatbereiche 38 und 42, die erste und zweite Endbereiche 124, 126 definieren, wurden durch anodische Oxidation mit der jeweiligen Beschichtung 92 beziehungsweise 94 versehen. Im nächsten Schritt wurde die Schutzschicht 96 am dritten Implantatbereich 46 entfernt und das Implantat 10 nochmals anodisch oxidiert, jedoch mit einer niedrigeren Anodisierspannung U₂.

Das beschriebene medizinische Clipsystem 70 umfasst mindestens zwei medizinische Clips 14, von denen mindestens ein Clip 14 in Form eines permanenten Clips 30 und mindestens ein Clip 14 in Form eines temporären Clips 28 ausgebildet ist. Die Form des temporären Clips 28 entspricht der Form und der Größe des permanenten Clips 30. Die Zuordnung der temporären Clips 28 zu den permanenten Clips 30 erfolgt wie beschrieben dadurch, dass der temporäre Clip 28 einen Clipbereich 48 aufweist, welcher farbig entsprechend dem permanenten Clip 30 eingefärbt ist.

In Verbindung mit den Figuren 1 bis 23 sind Ausführungsbeispiele medizinischer Implantate 10 in Form von Clips 14 beschrieben.

Die farbige Gestaltung von Oberflächen der Implantate 10 wird auch zur entsprechenden Kennzeichnung von Ausführungsbeispielen temporärer und permanenter Implantate eingesetzt, die in Form von Schrauben ausgebildet sind. Ein Implantatbereich, beispielsweise der dritte Implantatbereich 46, kann bei diesen Schrauben zur Kennzeichnung und Kodierung insbesondere einer Dicke, einer Länge oder des jeweiligen Schraubentyps genutzt werden.

Eine Zuordnung von Farben zur jeweiligen Eigenschaft des Implantats 10 ist bei der Herstellung des Implantats 10 frei wählbar.

### Bezugszeichenliste

- 10: Implantat
- 12: Applikationsinstrument
- 14: Clip
- 16: Clipanlegezange
- 18: Werkzeugelement
- 20: Branche
- 22: Schaft
- 24: Klemmarm
- 26: Aneurysmenclip
- 28: temporärer Clip
- 30: permanenter Clip
- 32: vorspannendes Element
- 34: Schlussbereich
- 36: Clipabschnitt
- 38: erster Implantatbereich
- 40: erster Clipbereich
- 42: zweiter Implantatbereich
- 44: zweiter Clipbereich
- 46: dritter Implantatbereich
- 48: dritter Clipbereich
- 50: distales Ende
- 52: proximales Ende
- 54: erster Übergangsbereich
- 56: zweiter Übergangsbereich
- 58: erster geometrisch definierter Bereich
- 60: zweiter geometrisch definierter Bereich
- 62: Gesamtlänge
- 64: Beschichtung
- 66: Beschichtung
- 68: Beschichtung
- 70: medizinisches Clipsystem
- 72: Beschichtungseinrichtung
- 74: Gleichspannungsquelle
- 76: Elektrolysezelle
- 78: Elektrolyt
- 80: Pluspol (Anode)
- 82: Anodisierspannung
- 84: Potentiometer
- 86: Behälter
- 88: Minuspol (Kathode)
- 90: Beschichtung
- 92: Beschichtung
- 94: Beschichtung
- 96: Schutzschicht
- 98: Schutzschicht
- 100: Dicke
- 102: Oxidschicht
- 104: Oxidschicht
- 106: Oxidschicht
- 108: Dicke
- 110: Werkstoff
- 112: Licht
- 114: Auftreffpunkt
- 116: Wellenfront
- 118: Punkt
- 120: Wellenfront
- 122: Austrittspunkt
- 124: erster Endbereich
- 126: zweiter Endbereich

## Patentansprüche

1. Medizinischer Clip (14), insbesondere in Form eines Aneurysmenclips (26), welcher zwei Klemmarme (24) und ein vorspannendes Element (32) umfasst, wobei jeweils ein Klemmarm (24) an einem freien Ende des vorspannenden Elements (32) angeordnet oder ausgebildet ist und wobei die zwei Klemmarme (24) in einer Grundstellung aneinander anliegen und entgegen der Wirkung des vorspannenden Elements (32) in eine Öffnungsstellung voneinander weg bewegbar sind, wobei mindestens ein Teil einer Oberfläche des Clips (14) farbig ausgebildet ist, wobei der Clip (14) mindestens drei voneinander räumlich getrennte Clipbereiche (40, 44, 48) definiert und benachbarte Clipbereiche (40, 44, 48) der mindestens drei Clipbereiche (40, 44, 48) unterschiedlich farbig ausgebildet sind, wobei ein erster Clipbereich (40) der mindestens drei Clipbereiche (40, 44, 48) freie Enden der zwei Klemmarme (24) umfasst, und wobei ein zweiter Clipbereich (44) der mindestens drei Clipbereiche (40, 44, 48) das vorspannende Element (32) oder einen Teil desselben umfasst, **dadurch gekennzeichnet, dass** mindestens ein dritter Clipbereich (48) der mindestens drei Clipbereiche (40, 44, 48) zwischen dem ersten Clipbereich (40) und dem zweiten Clipbereich (44) angeordnet oder ausgebildet ist.

2. Medizinischer Clip nach Anspruch 1, **dadurch gekennzeichnet, dass**
a) mindestens zwei der mindestens drei Clipbereiche (40, 44, 48) gleichfarbig ausgebildet sind und dass diese mindestens zwei Clipbereiche (40, 44, 48) nicht direkt aneinander angrenzen
und/oder
b) der erste Clipbereich (40) und der zweite Clipbereich (44) heller gefärbt sind als der mindestens eine dritte Clipbereich (48).

3. Medizinischer Clip nach einem der voranstehenden Ansprüche, **dadurch gekennzeichnet, dass**
a) mindestens einer der mindestens drei Clipbereiche (40, 44, 48) in einer der Farben Grün, Blau, Violett, Gelb oder goldfarbig gefärbt ist,
wobei insbesondere entweder die Oberfläche des mindestens einen dritten Clipbereichs (48) gelb oder goldfarbig ausgebildet ist oder dass die Oberflächen des ersten Clipbereichs (40) und des zweiten Clipbereichs (44) gelb oder goldfarbig ausgebildet sind,
und/oder
b) zwei aneinander angrenzende Clipbereiche (40, 44, 48) der mindestens drei Clipbereiche (40, 44, 48) einen farbigen Übergangsbereich (54, 56) der Oberfläche definieren und dass der farbige Übergangsbereich (54, 56) an einem geometrisch definierten Bereich (58, 60) des Clips (14) angeordnet oder ausgebildet ist, insbesondere in einem geometrisch definierten Bereich (58, 60), dessen Querschnitt auf einer Bereichslänge, welche mindestens etwa 5%, insbesondere etwa 10%, einer Gesamtlänge (62) des Clips (14) entspricht, konstant ist oder sich konisch verjüngt oder erweitert.

4. Medizinischer Clip nach einem der voranstehenden Ansprüche, **dadurch gekennzeichnet, dass** die Oberfläche mindestens eines Clipbereichs (40, 44, 48), insbesondere aller Clipbereiche (40, 44, 48), der mindestens drei Clipbereiche (40, 44, 48) durch eine Beschichtung (90, 92, 94) farbig ausgebildet ist,
wobei insbesondere
a) eine Schichtdicke (100, 108) der Beschichtung (90, 92, 94) in einem Bereich von etwa 10 nm bis etwa 500 nm liegt, insbesondere in einem Bereich von etwa 20 nm bis etwa 200 nm,
und/oder
b) die Beschichtung (64, 66, 68, 90, 92, 94) in Form einer durch anodische Oxidation ausgebildeten Oxidschicht (102, 104, 106) ausgebildet ist,
wobei insbesondere die Oxidschicht (102, 104, 106) einen Interferenzfilter bildet und dass eine Farbwirkung der Oxidschicht (102, 104, 106) von einer Schichtdicke (100, 108) derselben abhängt,
und/oder
c) Clipbereiche (40, 44, 48) mit gleichfarbig ausgebildeter Oberfläche mit einer identischen Beschichtung (64, 66, 68, 90, 92, 94) versehen sind
und/oder
d) die Beschichtung (64, 66, 68, 90, 92, 94) Gold enthält oder aus Gold besteht,
wobei insbesondere die Beschichtung (64, 66, 68, 90, 92, 94) in Form einer Tauchbeschichtung ausgebildet ist.

5. Medizinischer Clip nach einem der voranstehenden Ansprüche, **dadurch gekennzeichnet, dass** mindestens ein Clipbereich (40, 44, 48) der mindestens drei Clipbereiche (40, 44, 48) unbeschichtet ist.

6. Medizinischer Clip nach einem der voranstehenden Ansprüche, **dadurch gekennzeichnet, dass** der Clip (14) aus einem körperverträglichen Metall, insbesondere aus Titan, Aluminium, Tantal oder einer metallischen Legierung, ausgebildet ist,
wobei insbesondere die metallische Legierung Kobalt und/oder Chrom und/oder Nickel enthält.

7. Medizinisches Clipsystem (70) umfassend mindestens zwei medizinische Clips (14), wobei mindestens ein Clip (14) in Form eines permanenten Clips (30) ausgebildet ist, dessen Oberfläche farbig, insbesondere vollständig, ausgebildet ist, und wobei mindestens ein Clip (14), welcher in Form und/oder Größe dem permanenten Clip (30) entspricht, in Form eines temporären Clips (28) ausgebildet ist, dessen Oberfläche mindestens teilweise farbig entsprechend dem permanenten Clip (30) und mindestens teilweise farbig zur Kodierung als temporärer Clip (28) ausgebildet ist, **dadurch gekennzeichnet, dass** der temporäre Clip (28) in Form eines medizinischen Clips (14) nach einem der voranstehenden Ansprüche ausgebildet ist,
wobei insbesondere
a) die Farbe der Oberfläche des permanenten Clips (30) eine Kodierung für Form und/oder Größe des permanenten Clips (30) bildet
und/oder
b) jeder der mindestens zwei medizinischen Clips (14) zwei Klemmarme (24) und ein vorspannendes Element (32) umfasst, wobei jeweils ein Klemmarm (24) an einem freien Ende des vorspannenden Elements (32) angeordnet oder ausgebildet ist und wobei die zwei Klemmarme (24) in einer Grundstellung aneinander anliegen und entgegen der Wirkung des vorspannenden Elements (32) in eine Öffnungsstellung voneinander weg bewegbar sind,
und/oder
c) der permanente Clip (30) und der temporäre Clip (28) bis auf die Farbgebung identisch ausgebildet sind
und/oder
d) das Clipsystem (70) mindestens zwei sich in Größe und/oder Form unterscheidende permanente Clips (28, 30) umfasst.

8. Verfahren zum Herstellen eines medizinischen Implantats (10), nämlich eines medizinischen Clips (14), wobei eine Implantatoberfläche des medizinischen Implantats (10) farbig ausgebildet wird, wobei am Implantat (10) mindestens drei voneinander räumlich getrennte Implantatbereiche (38, 42, 46) definiert werden und benachbarte Implantatbereiche (38, 42, 46) der mindestens drei Implantatbereiche (38, 42, 46) unterschiedlich farbig ausgebildet werden, wobei ein erster Implantatbereich (38) der mindestens drei Implantatbereiche (38, 42, 46) durch einen ersten Endbereich (124) des Implantats (10) definiert wird, und wobei ein zweiter Implantatbereich (42) der mindestens drei Implantatbereiche (38, 42, 46) durch einen zweiten Endbereich (126) des Implantats (10) definiert wird, **dadurch gekennzeichnet, dass** mindestens ein dritter Implantatbereich (46) der mindestens drei Implantatbereiche (38, 42, 46) zwischen dem ersten Implantatbereich (38) und dem zweiten Implantatbereich (38) angeordnet oder ausgebildet wird.

9. Verfahren nach Anspruch 8, **dadurch gekennzeichnet, dass**
a) mindestens zwei der mindestens drei Implantatbereiche (38, 42, 46) gleichfarbig ausgebildet werden, wobei diese mindestens zwei Implantatbereiche (38, 42, 46) nicht direkt aneinander angrenzen
und/oder
b) zwei aneinander angrenzende Implantatbereiche (38, 42, 46) der mindestens drei Implantatbereiche (38, 42, 46) einen farbigen Übergangsbereich (54, 56) der Oberfläche definieren und dass der farbige Übergangsbereich (54, 56) an einem geometrisch definierten Bereich (58, 60) des Implantats (10) angeordnet oder ausgebildet wird, insbesondere in einem Bereich (58, 60), dessen Querschnitt auf einer Bereichslänge, welche mindestens etwa 5%, insbesondere etwa 10%, einer Gesamtlänge (62) des Implantats (10) entspricht, konstant ist oder sich konisch verjüngt oder erweitert.

10. Verfahren nach Anspruch 9 oder 10, **dadurch gekennzeichnet, dass** mindestens einer der mindestens drei Implantatbereiche (38, 42, 46) in einer der Farben grün, blau, violett, gelb oder goldfarbig gefärbt wird, wobei insbesondere
a) der erste Implantatbereich (38) und der zweite Implantatbereich (42) heller gefärbt sind als der mindestens eine dritte Implantatbereich (46)
und/oder
b) entweder die Oberfläche des mindestens einen dritten Implantatbereichs (46) gelb oder goldfarbig ausgebildet wird oder dass die Oberflächen des ersten Implantatbereichs (38) und des zweiten Implantatbereichs (42) gelb oder goldfarbig ausgebildet werden.

11. Verfahren nach einem der Ansprüche 8 bis 10, **dadurch gekennzeichnet, dass** die Oberfläche mindestens eines Implantatbereichs (38, 42, 46), insbesondere aller Implantatbereiche (38, 42, 46), der mindestens drei Implantatbereiche (38, 42, 46) mit einer farbigen Beschichtung (64, 66, 68, 90, 92, 94) versehen wird,
wobei insbesondere
a) die Beschichtung (64, 66, 68, 90, 92, 94) mit einer Schichtdicke (100, 108) in einem Bereich von etwa 10 nm bis etwa 500 nm ausgebildet wird, insbesondere in einem Bereich von etwa 20 nm bis etwa 200 nm,
und/oder
b) die Beschichtung (64, 66, 68, 90, 92, 94) in Form einer Oxidschicht (102, 104, 106) durch anodische Oxidation ausgebildet wird
und/oder
c) Implantatbereiche (38, 42, 46) mit gleichfarbig ausgebildeter Oberfläche mit einer identischen Beschichtung (64, 66, 68, 90, 92, 94) versehen werden
und/oder
d) die Beschichtung (64, 66, 68, 90, 92, 94) durch Vergolden, insbesondere durch ein Tauchverfahren, ausgebildet wird.

12. Verfahren nach einem der Ansprüche 8 bis 11, **dadurch gekennzeichnet, dass**
a) mindestens ein Implantatbereich (38, 42, 46) der mindestens drei Implantatbereiche (38, 42, 46) unbeschichtet belassen wird
und/oder
b) das Implantat (10) aus einem körperverträglichen Metall und/oder einem anodisch oxidierbaren Metall oder einer metallischen Legierung ausgebildet wird, wobei das Metall insbesondere Titan, Aluminium oder Tantal ist oder enthält.

13. Verfahren nach Anspruch 11 oder 12, **dadurch gekennzeichnet, dass** das Implantat (10) in einen Elektrolyt (78), insbesondere eine verdünnte Säure, eingebracht wird und dass zum anodischen Oxidieren das Implantat (10) mit der Anode (80) einer Gleichspannungsquelle (74) verbunden und mit einer Anodisierungsspannung U₁, U₂ beaufschlagt wird,
wobei insbesondere
a) zur Ausbildung unterschiedlicher Farben beim anodischen Oxidieren unterschiedliche Anodisierungsspannungen (U₁, U₂) an das Implantat (10) angelegt werden
und/oder
b) mindestens einer der mindestens drei Implantatbereiche (38, 42, 46) mit einer Schutzschicht (96, 98) bedeckt wird, dass dann das Implantat (10) mit einer ersten Anodisierungsspannung U₁ anodisch oxidiert wird zur Ausbildung einer ersten Oxidschicht (102) auf der Oberfläche des Implantats (10), die nicht mit der Schutzschicht (96, 98) bedeckt ist, dass die Schutzschicht (96, 98) entfernt wird, dass dann das Implantat (10) mit einer zweiten Anodisierungsspannung U₂ anodisch oxidiert wird zur Ausbildung einer zweiten Oxidschicht (104, 106) auf der Oberfläche des Implantats (10), wobei die erste Anodisierungsspannung U₂ größer ist als die zweite Anodisierungsspannung U₁,
wobei insbesondere die Schutzschicht (96, 98) durch eine Harzschicht ausgebildet wird.

14. Verfahren nach einem der Ansprüche 8 bis 13, **dadurch gekennzeichnet, dass** der medizinische Clip (14) mit zwei Klemmarmen (24) und einem vorspannenden Element (32) ausgebildet wird, dass jeweils ein Klemmarm (24) an einem freien Ende des vorspannenden Elements (32) angeordnet oder ausgebildet wird und dass die zwei Klemmarme (24) in einer Grundstellung aneinander anliegen und entgegen der Wirkung des vorspannenden Elements (32) in eine Öffnungsstellung voneinander weg bewegbar sind.

15. Verfahren nach Anspruch 14, **dadurch gekennzeichnet, dass** mindestens einer der mindestens drei Implantatbereiche (38, 42, 46) mit einer Schutzschicht (96, 98) bedeckt wird und vor dem Abdecken eines der mindestens drei Implantatbereiche (38, 42, 46) mit der Schutzschicht (96, 98) der Clip (14) geöffnet wird.

## Claims

1. Medical clip (14), in particular in the form of an aneurysm clip (26), which comprises two clamping arms (24) and a biasing element (32), wherein in each case a clamping arm (24) is arranged or formed on a free end of the biasing element (32), and wherein the two clamping arms (24) in a basic position abut against one another and are movable away from one another against the action of the biasing element (32) into an open position, wherein at least part of a surface of the clip (14) is of colored configuration, wherein the clip (14) defines at least three clip regions (40, 44, 48) that are spatially separate from one another and adjacent clip regions (40, 44, 48) of the at least three clip regions (40, 44, 48) are of differently colored configuration, wherein a first clip region (40) of the at least three clip regions (40, 44, 48) comprises free ends of the two clamping arms (24), and wherein a second clip region (44) of the at least three clip regions (40, 44, 48) comprises the biasing element (32) or part thereof, **characterized in that** at least one third clip region (48) of the at least three clip regions (40, 44, 48) is arranged or formed between the first clip region (40) and the second clip region (44).

2. Medical clip in accordance with Claim 1, **characterized in that**
a) at least two of the at least three clip regions (40, 44, 48) are of identically colored configuration and **in that** these at least two clip regions (40, 44, 48) do not directly adjoin one another
and/or
b) the first clip region (40) and the second clip region (44) are lighter colored than the at least one third clip region (48).

3. Medical clip in accordance with any one of the preceding Claims,
**characterized in that**
a) at least one of the at least three clip regions (40, 44, 48) is colored in one of the colors green, blue, violet, yellow, or golden,
wherein, in particular, either the surface of the at least one third clip region (48) is of yellow or golden configuration, or **in that** the surfaces of the first clip region (40) and the second clip region (44) are of yellow or golden configuration,
and/or
b) two adjoining clip regions (40, 44, 48) of the at least three clip regions (40, 44, 48) define a colored transition region (54, 56) of the surface and **in that** the colored transition region (54, 46) is arranged or formed on a geometrically defined region (58, 60) of the clip (14), in particular in a geometrically defined region (58, 60), the cross section of which is constant or conically tapers or widens on a region length, which corresponds to at least about 5%, in particular about 10%, of a total length (62) of the clip (14).

4. Medical clip in accordance with any one of the preceding Claims, **characterized in that** the surface of at least one clip region (40, 44, 48), in particular all clip regions (40, 44, 48), of the at least three clip regions (40, 44, 48) is of colored configuration by way of a coating (90, 92, 94),
wherein, in particular,
a) a layer thickness (100, 108) of the coating (90, 92, 94) is in a range of about 10 nm to about 500 nm, in particular in a range of about 20 nm to about 200 nm,
and/or
b) the coating (64, 66, 68, 90, 92, 94) is configured in the form of an oxide layer (102, 104, 106) formed by anodic oxidation, wherein, in particular, the oxide layer (102, 104, 106) forms an interference filter, and **in that** a color effect of the oxide layer (102, 104, 106) depends on a layer thickness (100, 108) thereof,
and/or
c) clip regions (40, 44, 48) with surfaces of identically colored configuration are provided with an identical coating (64, 66, 68, 90, 92, 94),
and/or
d) the coating (64, 66, 68, 90, 92, 94) contains gold or consists of gold,
wherein, in particular, the coating (64, 66, 68, 90, 92, 94) is configured in the form of a dip coating.

5. Medical clip in accordance with any one of the preceding Claims,
**characterized in that** at least one clip region (40, 44, 48) of the at least three clip regions (40, 44, 48) is uncoated.

6. Medical clip in accordance with any one of the preceding Claims,
**characterized in that** the clip (14) is made of a biocompatible metal, in particular of titanium, aluminum, tantalum, or a metallic alloy,
wherein, in particular, the metallic alloy contains cobalt and/or chromium and/or nickel.

7. Medical clip system (70) comprising at least two medical clips (14), wherein, in particular, at least one clip (14) is configured in the form of a permanent clip (30), the surface of which is of, in particular completely, colored configuration, and wherein at least one clip (14), which corresponds in shape and/or size with the permanent clip (30), is configured in the form of a temporary clip (28), the surface of which is configured at least partially corresponding in color to the permanent clip (30) and at least partially colored for coding as a temporary clip (28), **characterized in that** the temporary clip (28) is configured in the form of a medical clip (14) in accordance with any one of the preceding Claims, wherein, in particular,
a) the color of the surface of the permanent clip (30) forms a coding for the shape and/or size of the permanent clip (30)
and/or
b) each of the at least two medical clips (14) comprises two clamping arms (24) and a biasing element (32), wherein in each case a clamping arm (24) is arranged or formed on a free end of the biasing element (32), and wherein the two clamping arms (24) in a basic position abut against one another and are movable away from one another against the action of the biasing element (32) into an open position,
and/or
c) the permanent clip (30) and the temporary clip (28) are of identical configuration except for the coloring
and/or
d) the clip system (70) comprises at least two permanent clips (28, 30) differing in size and/or shape.

8. Method for producing a medical implant (10), namely a medical clip (14), wherein an implant surface of the medical implant (10) is of colored configuration, wherein at least three implant regions (38, 42, 46) that are spatially separate from one another are defined on the implant (10) and in that adjacent implant regions (38, 42, 46) of the at least three implant regions (38, 42, 46) are of differently colored configuration, wherein a first implant region (38) of the at least three implant regions (38, 42, 46) is defined by a first end region (124) of the implant (10), and wherein a second implant region (42) of the at least three implant regions (38, 42, 46) is defined by a second end region (126) of the implant (10), **characterized in that** at least one third implant region (46) of the at least three implant regions (38, 42, 46) is arranged or formed between the first implant region (38) and the second implant region (38).

9. Method in accordance with Claim 8, **characterized in that**
a) at least two of the at least three implant regions (38, 42, 46) are of identically colored configuration, wherein these at least two implant regions (38, 42, 46) do not directly adjoin one another,
and/or
b) two adjoining implant regions (38, 42, 46) of the at least three implant regions (38, 42, 46) define a colored transition region (54, 56) of the surface and **in that** the colored transition region (54, 56) is arranged or formed on a geometrically defined region (58, 60) of the implant (10), in particular in a region (58, 60), the cross section of which is constant or conically tapers or widens on a region length, which corresponds to at least about 5%, in particular about 10%, of a total length (62) of the implant (10).

10. Method in accordance with Claim 9 or 10, **characterized in that** at least one of the at least three implant regions (38, 42, 46) is colored in one of the colors green, blue, violet, yellow, or golden,
and/or
wherein, in particular,
a) the first implant region (38) and the second implant region (42) are lighter colored than the at least one third implant region (46)
and/or
b) either the surface of the at least one third implant region (46) is of yellow or golden configuration, or **in that** the surfaces of the first implant region (38) and the second implant region (42) are of yellow or golden configuration.

11. Method in accordance with any one of Claims 8 to 10, **characterized in that** the surface of at least one implant region (38, 42, 46), in particular of all implant regions (38, 42, 46), of the at least three implant regions (38, 42, 46) is provided with a colored coating (64, 66, 68, 90, 92, 94), wherein, in particular,
a) the coating (64, 66, 68, 90, 92, 94) is configured with a layer thickness (100, 108) in a range of about 10 nm to about 500 nm, in particular in a range of about 20 nm to about 200 nm,
and/or
b) the coating (64, 66, 68, 90, 92, 94) is configured in the form of an oxide layer (102, 104, 106) formed by anodic oxidation,
and/or
c) implant regions (38, 42, 46) with surfaces of identically colored configuration are provided with an identical coating (64, 66, 68, 90, 92, 94),
and/or
d) the coating (64, 66, 68, 90, 92, 94) is formed by gold plating, in particular by way of a dip process.

12. Method in accordance with any one of Claims 8 to 11, **characterized in that**
a) at least one implant region (38, 42, 46) of the at least three implant regions (38, 42, 46) are left uncoated,
and/or
b) the implant (10) is made of a biocompatible metal and/or an anodically oxidizable metal or a metallic alloy, wherein the metal is or contains titanium, aluminum, or tantalum.

13. Method in accordance with Claim 11 or 12, **characterized in that** the implant (10) is introduced into an electrolyte (78), in particular a diluted acid, and **in that** the implant (10) is connected to the anode (80) of a direct voltage source (74) and is subjected to an anodizing voltage U₁, U₂ for the purpose of anodic oxidization,
wherein, in particular,
a) for forming different colors, different anodizing voltages (U₁, U₂) are applied to the implant (10) during the anodic oxidation,
and/or
b) at least one of the at least three implant regions (38, 42, 46) is covered with a protective layer (96, 98), **in that** the implant (10) is then anodically oxidized with a first anodizing voltage U₁ for forming a first oxide layer (102) on the surface of the implant (10) that is not covered with the protective layer (96, 98), **in that** the protective layer (96, 98) is removed, **in that** the implant (10) is then anodically oxidized with a second anodizing voltage U₂ for forming a second oxide layer (104, 106) on the surface of the implant (10), wherein the first anodizing voltage U₂ is greater than the second anodizing voltage U₁,
wherein, in particular, the protective layer (96, 98) is formed by a resin layer.

14. Method in accordance with any one of Claims 8 to 13, **characterized in that** the medical clip (14) is configured with two clamping arms (24) and a biasing element (32), **in that** in each case a clamping arm (24) is arranged or formed on a free end of the biasing element (32), and **in that** the two clamping arms (24) in a basic position abut against one another and are movable away from one another against the action of the biasing element (32) into an open position.

15. Method in accordance with Claim 14, **characterized in that** at least one of the at least three implant regions (38, 42, 46) is covered with a protective layer and **in that** the clip is opened before covering one of the at least three implant regions (38, 42, 46) with the protective layer (96, 98).

## Revendications

1. Clip médical (14), en particulier sous la forme d'un clip d'anévrisme (26) qui comprend deux bras de serrage (24) et un élément de précontrainte (32), un bras de serrage (24) étant agencé ou formé à une extrémité libre de l'élément de précontrainte (32) et les deux bras de serrage (24) reposant l'un contre l'autre dans une position de base et pouvant être écartés l'un de l'autre à l'encontre de l'action de l'élément de précontrainte (32), au moins une partie d'une surface du clip (14) étant colorée, le clip (14) définissant au moins trois zones de clip (40, 44, 48) séparées les unes des autres dans l'espace et les zones de clip adjacentes (40, 44, 48) parmi les au moins trois zones de clip (40, 44, 48) de couleurs différentes, une première zone de clip (40) parmi les au moins trois zones de clip (40, 44, 48) comprenant les extrémités libres des deux bras de serrage (24), et une deuxième zone de clip (44) parmi les au moins trois zones de clip (40, 44, 48) comprenant l'élément de précontrainte (32) ou une partie de celui-ci, **caractérisé en ce qu'**au moins une troisième zone de clip (48) parmi les au moins trois zones de clip (40, 44, 48) est agencée ou formée entre la première zone de clip (40) et la deuxième zone de clip (44).

2. Clip médical selon la revendication 1, **caractérisé en ce que**
a) au moins deux parmi les au moins trois zones de clip (40, 44, 48) sont de même couleur et **en ce que** ces au moins deux zones de clip (40, 44, 48) ne sont pas directement adjacentes l'une à l'autre
et/ou
b) la première zone de clip (40) et la deuxième zone de clip (44) sont de couleur plus claire que l'au moins une troisième zone de clip (48).

3. Clip médical selon l'une des revendications précédentes, **caractérisé en ce que**
a) au moins l'une parmi les au moins trois zones de clip (40, 44, 48) est de couleur verte, bleue, violette, jaune ou dorée, dans lequel, en particulier, soit la surface de l'au moins une troisième zone de clip (48) est de couleur jaune ou dorée, soit les surfaces de la première zone de clip (40) et de la deuxième zone de clip (44) sont de couleur jaune ou dorée,
et/ou
b) deux zones de clip adjacentes (40, 44, 48) parmi les au moins trois zones de clip (40, 44, 48) définissent une zone de transition colorée (54, 56) de la surface et la zone de transition colorée (54, 56) est agencée ou formée dans une zone géométriquement définie (58, 60) du clip (14), en particulier dans une zone géométriquement définie (58, 60) dont la section transversale est constante ou se rétrécit ou s'élargit de manière conique sur une longueur de zone qui correspond à au moins environ 5 %, en particulier environ 10 %, d'une longueur totale (62) du clip (14).

4. Clip médical selon l'une des revendications précédentes, **caractérisé en ce que** la surface d'au moins une zone de clip (40, 44, 48), en particulier de toutes les zones de clip (40, 44, 48), parmi les au moins trois zones de clip (40, 44, 48) est colorée par un revêtement (90, 92, 94),
dans lequel, en particulier,
a) une épaisseur de couche (100, 108) du revêtement (90, 92, 94) se situe dans une plage d'environ 10 nm à environ 500 nm, en particulier dans une plage d'environ 20 nm à environ 200 nm,
et/ou
b) le revêtement (64, 66, 68, 90, 92, 94) est réalisé sous la forme d'une couche d'oxyde (102, 104, 106) formée par oxydation anodique,
dans lequel, en particulier, la couche d'oxyde (102, 104, 106) forme un filtre interférentiel et dans lequel l'effet de couleur de la couche d'oxyde (102, 104, 106) dépend de l'épaisseur (100, 108) de celle-ci,
et/ou
c) les zones de clip (40, 44, 48) avec une surface de même couleur sont pourvues d'un revêtement identique (64, 66, 68, 90, 92, 94)
et/ou
d) le revêtement (64, 66, 68, 90, 92, 94) contient de l'or ou est constitué d'or,
dans lequel, en particulier, le revêtement (64, 66, 68, 90, 92, 94) est réalisé sous la forme d'un revêtement par immersion.

5. Clip médical selon l'une des revendications précédentes, **caractérisé en ce qu'**au moins une zone de clip (40, 44, 48) parmi les au moins trois zones de clip (40, 44, 48) est dépourvue de revêtement.

6. Clip médical selon l'une des revendications précédentes, **caractérisé en ce que** le clip (14) est réalisé dans un métal biocompatible, en particulier en titane, en aluminium, en tantale ou dans un alliage métallique, dans lequel, en particulier, l'alliage métallique contient du cobalt et/ou du chrome et/ou du nickel.

7. Système de clips médicaux (70) comprenant au moins deux clips médicaux (14), au moins un clip (14) étant conçu sous la forme d'un clip permanent (30) dont la surface est colorée, en particulier entièrement, et au moins un clip (14), qui correspond au clip permanent (30) en termes de forme et/ou de taille et est conçu sous la forme d'un clip temporaire (28) dont la surface est au moins partiellement colorée de manière à correspondre au clip permanent (30) et au moins partiellement colorée pour être codée en tant que clip temporaire (28), **caractérisé en ce que** le clip temporaire (28) est conçu sous la forme d'un clip médical (14) selon l'une des revendications précédentes, dans lequel, en particulier,
a) la couleur de la surface du clip permanent (30) constitue un code pour la forme et/ou la taille du clip permanent (30)
et/ou
b) chacun des au moins deux clips médicaux (14) comprend deux bras de serrage (24) et un élément de précontrainte (32), dans lequel un bras de serrage (24) est agencé ou formé à une extrémité libre de l'élément de précontrainte (32) et dans lequel les deux bras de serrage (24) sont adjacents l'un à l'autre dans une position de base et peuvent être écartés l'un de l'autre dans une position d'ouverture à l'encontre de l'action de l'élément de précontrainte (32),
et/ou
c) le clip permanent (30) et le clip temporaire (28) sont identiques, à l'exception de leur couleur,
et/ou
d) le système de clips (70) comprend au moins deux clips permanents (28, 30) de taille et/ou de forme différentes.

8. Procédé de fabrication d'un implant médical (10), à savoir un clip médical (14), dans lequel une surface de l'implant médical (10) est colorée, au moins trois zones d'implant (38, 42, 46) séparées les unes des autres dans l'espace étant définies sur l'implant (10) et les zones d'implant adjacentes (38, 42, 46) parmi les au moins trois zones d'implant (38, 42, 46) étant colorées différemment, une première zone d'implant (38) parmi les au moins trois zones d'implant (38, 42, 46) étant définie par une première zone d'extrémité (124) de l'implant (10), et une deuxième zone d'implant (42) parmi les au moins trois zones d'implant (38, 42, 46) étant définie par une deuxième zone d'extrémité (126) de l'implant (10), **caractérisé en ce qu'**au moins une troisième zone d'implant (46) parmi les au moins trois zones d'implant (38, 42, 46) est agencée ou formée entre la première zone d'implant (38) et la deuxième zone d'implant (38).

9. Procédé selon la revendication 8, **caractérisé en ce que**
a) au moins deux parmi les au moins trois zones d'implant (38, 42, 46) sont réalisées dans la même couleur, ces au moins deux zones d'implant (38, 42, 46) n'étant pas directement adjacentes l'une à l'autre
et/ou
b) deux zones d'implant (38, 42, 46) adjacentes parmi les au moins trois zones d'implant (38, 42, 46) définissent une zone de transition colorée (54, 56) de la surface et **en ce que** la zone de transition colorée (54, 56) est agencée ou formée dans une zone géométriquement définie (58, 60) de l'implant (10), en particulier dans une zone (58, 60) dont la section transversale est constante ou se rétrécit ou s'élargit de manière conique sur une longueur de zone qui correspond à au moins environ 5 %, en particulier environ 10 %, d'une longueur totale (62) de l'implant (10).

10. Procédé selon la revendication 9 ou 10, **caractérisé en ce qu'**au moins l'une parmi les au moins trois zones d'implant (38, 42, 46) est colorée dans l'une des couleurs parmi le vert, le bleu, le violet, le jaune ou le doré,
dans lequel, en particulier,
a) la première zone d'implant (38) et la deuxième zone d'implant (42) sont colorées dans une teinte plus claire que l'au moins une troisième zone d'implant (46)
et/ou
b) soit la surface de l'au moins une troisième zone d'implant (46) est de couleur jaune ou dorée, soit les surfaces de la première zone d'implant (38) et de la deuxième zone d'implant (42) sont de couleur jaune ou dorée.

11. Procédé selon l'une des revendications 8 à 10, **caractérisé en ce que** la surface de l'au moins une zone d'implant (38, 42, 46), en particulier de toutes les zones d'implant (38, 42, 46), parmi les au moins trois zones d'implant (38, 42, 46) est pourvue d'un revêtement coloré (64, 66, 68, 90, 92, 94),
où en particulier
a) le revêtement (64, 66, 68, 90, 92, 94) est réalisé avec une épaisseur de couche (100, 108) comprise entre environ 10 nm et environ 500 nm, en particulier entre environ 20 nm et environ 200 nm,
et/ou
b) le revêtement (64, 66, 68, 90, 92, 94) est formé sous la forme d'une couche d'oxyde (102, 104, 106) par oxydation anodique
et/ou
c) les zones d'implant (38, 42, 46) présentant une surface de même couleur sont pourvues d'un revêtement identique (64, 66, 68, 90, 92, 94)
et/ou
d) le revêtement (64, 66, 68, 90, 92, 94) est formé par dorure, en particulier par revêtement par immersion.

12. Procédé selon l'une des revendications 8 à 11, **caractérisé en ce que**
a) au moins une zone d'implant (38, 42, 46) parmi les au moins trois zones d'implant (38, 42, 46) est dépourvue de revêtement
et/ou
b) l'implant (10) est formé à partir d'un métal biocompatible et/ou d'un métal oxydable par anodisation ou d'un alliage métallique, le métal étant en particulier du titane, de l'aluminium ou du tantale ou en contenant.

13. Procédé selon la revendication 11 ou 12, **caractérisé en ce que** l'implant (10) est placé dans un électrolyte (78), en particulier un acide dilué et **en ce que**, pour l'oxydation anodique, l'implant (10) est relié à l'anode (80) d'une source de tension continue (74) et soumis à une tension d'anodisation U₁, U₂,
dans lequel, en particulier,
a) pour obtenir différentes couleurs lors de l'oxydation anodique, différentes tensions d'anodisation (U₁, U₂) sont appliquées à l'implant (10)
et/ou
b) au moins l'une parmi les au moins trois zones d'implant (38, 42, 46) est recouverte d'une couche protectrice (96, 98), que l'implant (10) est ensuite oxydé anodiquement avec une première tension d'anodisation U₁ pour former une première couche d'oxyde (102) sur la surface de l'implant (10) qui n'est pas recouverte par la couche protectrice (96, 98), que la couche protectrice (96, 98) est retirée, que l'implant (10) est ensuite oxydé par anodisation avec une deuxième tension d'anodisation U₂ pour former une deuxième couche d'oxyde (104, 106) sur la surface de l'implant (10), la première tension d'anodisation U₂ étant supérieure à la deuxième tension d'anodisation U₁,
dans lequel, en particulier, la couche protectrice (96, 98) est formée par une couche de résine.

14. Procédé selon l'une des revendications 8 à 13, **caractérisé en ce que** le clip médical (14) est formé avec deux bras de serrage (24) et un élément de précontrainte (32), **en ce qu'**un bras de serrage (24) est agencé ou formé à une extrémité libre de l'élément de précontrainte (32) et **en ce que** les deux bras de serrage (24) sont adjacents l'un à l'autre dans une position de base et peuvent être écartés l'un de l'autre dans une position d'ouverture à l'encontre de l'action de l'élément de précontrainte (32).

15. Procédé selon la revendication 14, **caractérisé en ce qu'**au moins l'une parmi les au moins trois zones d'implant (38, 42, 46) est recouverte d'une couche protectrice (96, 98) et **en ce que** le clip (14) est ouvert avant de recouvrir l'une parmi les au moins trois zones d'implant (38, 42, 46) avec la couche protectrice (96, 98).
